# EUROPEAN PATENT APPLICATION

(11) **EP 4 737 578 A2**
(43) Date of publication of application: **06.05.2026**
(21) Application number: 25212733.7
(22) Date of filing: 08.11.2019
(51) Int. Cl.: C12N 15/864

(54) **GENE THERAPY EMPLOYING GENOME EDITING WITH SINGLE AAV VECTOR**

(30) Priority: 08.11.2018 JP 2018210670
(62) Divisional of application: 19883277.6
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: NISHIGUCHI, Koji, Nagoya-shi, Aichi, 464-8601 (JP); NAKAZAWA, Toru, Sendai-shi, Miyagi, 980-8577 (JP); FUJITA, Kosuke, Nagoya-shi, Aichi, 464-8601 (JP)
(74) Representative: Hoffmann Eitle

(57) **Abstract**

An adeno-associated virus (AAV) vector for inserting a desired nucleic acid into a nucleic acid in a cell, wherein the nucleic acid in the cell comprises a region consisting of a first nucleotide sequence and a region consisting of a second nucleotide sequence in order in a direction from a 5' end to a 3' end, wherein the vector comprises a first gRNA target sequence, a region consisting of a first nucleotide sequence, the desired nucleic acid, a region consisting of a second nucleotide sequence, a second gRNA target sequence, a cell-specific promoter, a sequence encoding a Cas9 nuclease, an RNA polymerase III promoter, a sequence encoding a first gRNA recognizing the first gRNA target sequence and a sequence encoding a second gRNA recognizing the second gRNA target sequence, wherein the vector yields a nucleic acid fragment comprising a region consisting of a first nucleotide sequence, the desired nucleic acid and the region consisting of the second nucleotide sequence by the Cas9 nuclease, wherein a first nucleotide sequence in the nucleic acid in the cell and a first nucleotide sequence in the vector are linked by a microhomology-mediated joining and a second nucleotide sequence in the nucleic acid in the cell and a second nucleotide sequence in the vector are linked by a microhomology-mediated joining, thereby inserting the desired nucleic acid between the region consisting of the first nucleotide sequence and the region consisting of the second nucleotide sequence in the nucleic acid in the cell.

## Description

### [Technical Field]

The present invention relates to an adeno-associated virus (AAV) vector for inserting a desired nucleic acid into a corresponding nucleic acid in a cell, a method of introducing a nucleic acid, a cell including the desired nucleic acid and a therapeutic method for treating an ocular disease.

### [Background Art]

An adeno-associated virus (AAV) has been widely used in gene therapy and multiple successful examples of gene supplementation therapy using an AAV against an inherited retinal degeneration have recently been reported (Non Patent Literature 1). As for the therapeutic concept, it is very reasonable to aim to cure a disease by introducing a gene with a normal function to a target retinal cell using an AAV, aiming to compensate for the causal gene with decreased or lost function.

Although this therapeutic platform allows to target various genes, many of the frequent pathogenic genes causing retinal degeneration greatly exceed 4000 bp. This is a big problem since there is a restriction in the size of the gene (about 4000 bp or less) that can be effectively delivered by an AAV vector. For example, an EYS gene, which is a pathogenic gene with an overwhelmingly high frequency in Japanese patients with retinitis pigmentosa, is about 10000 bp. This gene cannot be treated by a conventional AAV gene supplementation therapy.

Meanwhile, in the recently developed PITCh (Precise Integration into Target Chromosome) method, a homology arm used to precisely insert a DNA fragment to a particular position on the genome is extremely short compared to a conventional homology arm. Thus, a use of this method greatly reduced the size of the element necessary for genome editing therapy (Patent Literature 1, Non Patent Literature 2).

### [Citation List]

### [Patent Literature]

[PTL 1] WO 2015/068785

### [Non Patent Literature]

[NPL 1] IOVS, September 2007, Vol.48, No.9
[NPL 2] NATURE PROTOCOL, Vol.11, No.1 published on line 17 December 2015, doi:10.1038/nprot.2015.140

### [Summary of Invention]

### [Technical Problem]

While there are reports of genome editing gene therapy using an AAV, none has been successful in accurately replacing a mutated sequence with a normal sequence by genome editing with a single AAV vector because the Cas9 gene required for genome editing is too large.

The problem to be solved by the present invention is to provide an AAV vector that can introduce a desired nucleic acid to a genome in a cell with a single vector, a method of introducing a nucleic acid using the AAV vector, a cell manufactured by the introduction method and a method for treating a disease using the AAV vector.

### [Solution to Problem]

While a conventional gene therapy assumes introducing a full-length gene by a vector, the inventors of the present invention found out that it is possible to prepare an AAV vector useful for gene therapy or mutation correction of a nucleic acid of a large gene by adopting a method of replacing only an abnormal sequence of a gene using genome editing (excise the abnormal sequence and insert a normal sequence), which led to the establishment of the present invention.

In other words, the present invention encompasses the subjects described in the following items.
Item 1. An adeno-associated virus (AAV) vector for inserting a desired nucleic acid into a nucleic acid in a cell,
   wherein the nucleic acid in the cell comprises a region consisting of a first nucleotide sequence and a region consisting of a second nucleotide sequence in order in a direction from a 5' end to a 3' end,
   wherein the vector comprises a first gRNA target sequence, a region consisting of a first nucleotide sequence, the desired nucleic acid, a region consisting of a second nucleotide sequence, a second gRNA target sequence, a cell-specific promoter, a sequence encoding a Cas9 nuclease, an RNA polymerase III promoter, a sequence encoding a first gRNA recognizing the first gRNA target sequence and a sequence encoding a second gRNA recognizing the second gRNA target sequence,
   wherein the vector yields a nucleic acid fragment comprising a region consisting of a first nucleotide sequence, the desired nucleic acid and the region consisting of the second nucleotide sequence by the Cas9 nuclease,
   wherein a first nucleotide sequence in the nucleic acid in the cell and a first nucleotide sequence in the vector are linked by a microhomology-mediated joining and a second nucleotide sequence in the nucleic acid in the cell and a second nucleotide sequence in the vector are linked by a microhomology-mediated joining, thereby inserting the desired nucleic acid between the region consisting of the first nucleotide sequence and the region consisting of the second nucleotide sequence into the nucleic acid in the cell.
Item 2. The vector of item 1, wherein the nucleic acid in the cell has a target nucleic acid sequence replaced with the desired nucleic acid between the region consisting of the first nucleotide sequence and the region consisting of the second nucleotide sequence,
   has a first gRNA target sequence recognized by the first gRNA of the AAV vector and a first PAM sequence between the region consisting of the first nucleotide sequence and the target nucleic acid sequence, and
   has a second gRNA target sequence recognized by the second gRNA of the AAV vector and a second PAM sequence between the target nucleic acid sequence and the region consisting of the second nucleotide sequence.
Item 3. The vector of item 2, wherein the vector satisfies any of the following (i) to (iv).
   (i) not having one or both of a sequence which is the same as the first gRNA target sequence and a sequence which is the same as the second gRNA target sequence between the region consisting of the first nucleotide sequence and the region consisting of the second nucleotide sequence,
   (ii) when there is a sequence which is the same as the first gRNA target sequence between the region consisting of the first nucleotide sequence and the region consisting of the second nucleotide sequence, the first PAM sequence adjacent to the first gRNA target sequence on the side close to the region consisting of the second nucleotide sequence being mutated so as to avoid cleavage of the first gRNA target sequence by a Cas9 nuclease,
   (iii) when there is a sequence which is the same as the second gRNA target sequence between the region consisting of the first nucleotide sequence and the region consisting of the second nucleotide sequence, the second PAM sequence adjacent to the second gRNA target sequence on the side close to the region consisting of the first nucleotide sequence being mutated so as to avoid cleavage of the second gRNA target sequence by a Cas9 nuclease, or
   (iv) both (ii) and (iii).
Item 4. The vector of any one of items 1 to 3, wherein the nucleic acid in the cell has a mutation type base between the region consisting of the first nucleotide sequence and the region consisting of the second nucleotide sequence, has a normal type base in which the desired nucleic acid of the vector corresponds to the mutation type base, wherein the mutation type base of the nucleic acid in the cell is replaced with the normal type base.
Item 5. The vector of any one of items 1 to 4, wherein a length of the cell-specific promoter is 200 bases or less.
Item 6. The vector of any one of items 1 to 5, wherein the cell-specific promoter is a promoter selected from rhodopsin kinase promoter, RPE65 promoter, Best1 promoter, mGluR6 promoter, cone arrestin promoter, CRALBP1 promoter, Chx10 promoter, rhodopsin promoter, cone opsin promoter, recoverin promoter, synapsin I promoter, myelin basic protein promoter, neuron-specific enolase promoter, calcium/calmodulin-dependent protein kinase II (CMKII) promoter, tubulin α I promoter, platelet-derived growth factor β chain promoter, glial fibrillary acidic protein (GFAP) promoter, L7 promoter and glutamic acid receptor delta 2 promoter; or a promoter having consecutive 50 to 150 base sequence of the promoter; or a promoter consisting of a base sequence that is 90% or more identical with the 50 to 150 base sequence.
Item 7. The vector of any one of items 1 to 6, wherein a length of a base of the first nucleotide sequence in the vector is 5 to 40 and a length of a base of the second nucleotide sequence in the vector is 5 to 40.
Item 8. A kit for inserting a desired nucleic acid into a nucleic acid in a cell, comprising the vector of any one of items 1 to 7.
Item 9. A method of inserting a desired nucleic acid into a nucleic acid in a cell, comprising the step of introducing the vector of any one of items 1 to 7 to a cell.
Item 10. A method of manufacturing a cell comprising a desired nucleic acid, comprising:
   the step of introducing the vector of any one of items 1 to 7 to a cell; and
   the step of inserting a desired nucleic acid into the nucleic acid in the cell.
Item 11. A method for treating a disease in vitro or in a non-human animal, comprising:
   the step of introducing the vector of any of items 1 to 7 to a cell; and
   the step of inserting a desired nucleic acid into the nucleic acid in the cell.
Item 12. The method of item 11, wherein the cell is an ocular cell.
Item 13. A therapeutic composition for treating a disease, comprising the vector of any one of items 1 to 7.

### [Advantageous Effects of Invention]

The AAV vector of the present invention can be widely applied to treat various genetic diseases or correct mutations by targeting a target nucleic acid sequence in the genome in a cell.

### [Brief Description of Drawings]

[Figure 1] A figure showing a sequence of a rhodopsin kinase promoter for various types of mammals.
[Figure 2] A microscopic image of retinal tissue showing the result of a reporter assay using each type of promoter. Stimulus intensity: intensity of stimulation.

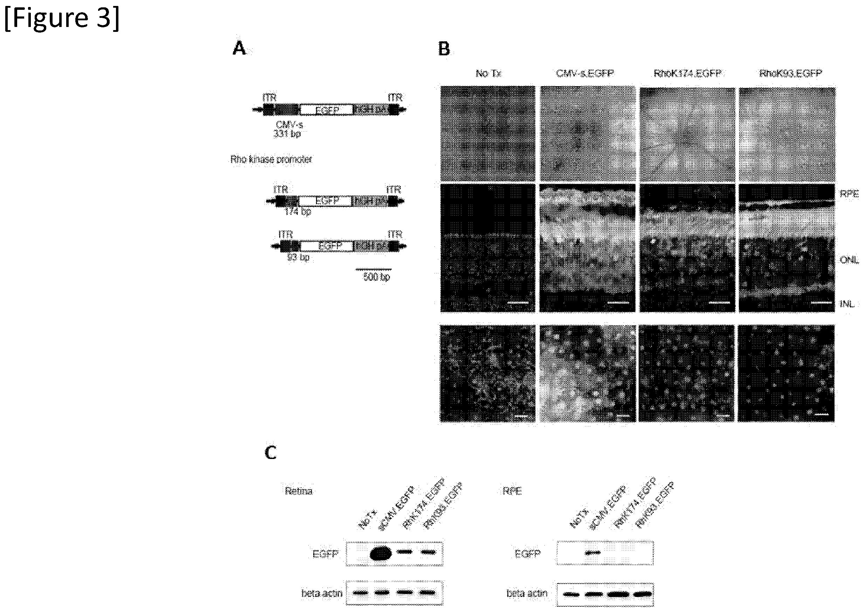

[Figure 3] Identification of a promoter that allows photoreceptor cell-specific expression. (A) Schematic view of an AAV construct. CMV-s: truncated CMV promoter, ITR: inverted terminal repeat, hGh pA: human growth hormone poly A signal. (B) Images of an in vivo reporter assay in a wild-type mouse in the first week after AAV injection. The upper panels are images of an in vivo confocal laser ophthalmoscope. The middle panels are images of a cryosection of a retina. The scale bar shows 20 µm. No Tx: no treatment (no administration of an AAV), RPE: retinal pigment epithelial layer, ONL: outer nuclear layer, INL: inner nuclear layer. The lower panels are images of a flat mount of the retinal pigment epithelial layer. The scale bar shows 20 µm. (C) Western blot of the retina (left side) and the retinal pigment epithelium (RPE) using an anti-EGFP antibody. β-actin was used as an internal control.
[Figure 4] Design of All-in-one AAV vector.

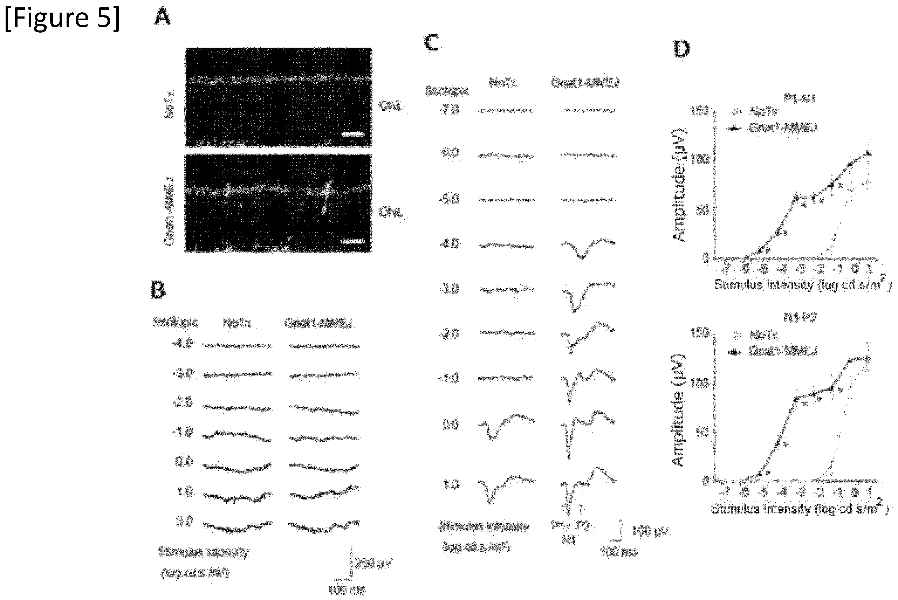

[Figure 5] Recovery of light response of rod photoreceptors in an adult Pde6^{cpfl1/cpfl1}Gnat^{1IRD2/IRD2} mouse after MMEJ-mediated normalization of Gnat1 mutation by an AAV (AAV-MMEJ-Gnat1). (A) Images a retinal section showing immune reaction against Gnat1 (green color) 3 weeks after AAV injection. The arrows show Gnat1-positive photoreceptors. The scale bar shows 20 µm. (B) Traces of an electroretinogram (ERG) of a Pde6 ^{cpfl1/cpfl1}Gnat1 ^{IRD2/IRD2} mouse 1 week after AAV-MMEJ-Gnat1 injection (Gnat1-MMEJ). (C) Traces of fVEP (flash visually evoked potential) measurements from an adult Pde6 ^{cpfl1/cpfl1}Gnat1 ^{IRD2/IRD2} mouse 3 weeks after AAV-MMEJ-Gnat1 injection (Gnat1-MMEJ). No Tx: no treatment, Stimulus intensity: intensity of stimulation. N=9. (D) Quantification of P1-N1 amplitude and N1-P2 amplitude in an adult Pde6 ^{cpfl1/cpfl1}Gnat1 ^{IRD2/IRD2} mouse 3 weeks after AAV-MMEJ-Gnat1 injection. Amplitude: amplitude, Stimulus intensity: intensity of stimulation. The data shows average value ± standard deviation. *P<0.05. No Tx: no treatment (no administration of an AAV), Student t-test was applied in (D).
[Figure 6] Immunohistological staining of cone photoreceptors. (A) EGFP, (B) M-opsin, (C) both EGFP and M-opsin.
[Figure 7] Schematic view of a gRNA design.
[Figure 8] A Table of gRNAs and their sequences.
[Figure 9] T7E1 assay for each gRNA. An expected DNA size is shown in an image of a representative gel from three independent replicates. A graph quantifying editing efficiency by (A) 5' gRNA, (B) 3' gRNA and each gRNA of (C) and (D).
[Figure 10] (A) Electrophoresis of a DNA fragment after cleavage of the genome by a gRNA pair. (B) A graph quantifying efficiency of two cleavage sites induced by a gRNA pair.

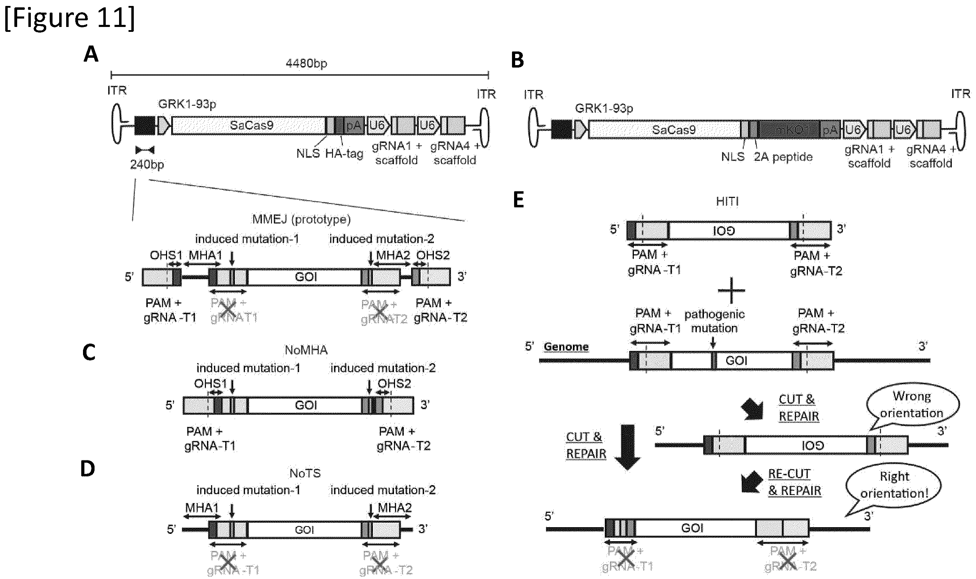

[Figure 11] (A) A design of an MMEJ mutation replacement vector with an enlarged image of the donor template, wherein the entire size is 4480bp, (B) a map of an MMEJ vector including a reporter for lineage tracing experimentation, wherein Sacas9 and Kusabirar-Orange 1 (mK01) are coupled to a 2A peptide, wherein the entire size is 5201bp, (C) a map of a donor template with no adjacent microhomology arm (NoMHA), (D) a map of a donor template with no adjacent gRNA target site (NoTS), (E) a map of a donor template for HITI (Homology-Independent Targeted Integration) mutation replacement vector, and a drawing explaining the HITI mutation replacement method. In this method, when subject gene (GOI) is inserted in a direction opposite to the adjacent gRNA target site of the donor template and GOI is inserted in the genome in a correct direction by NHEJ, the adjacent gRNA target site is disrupted and re-cleavage by SaCas9 is prevented.

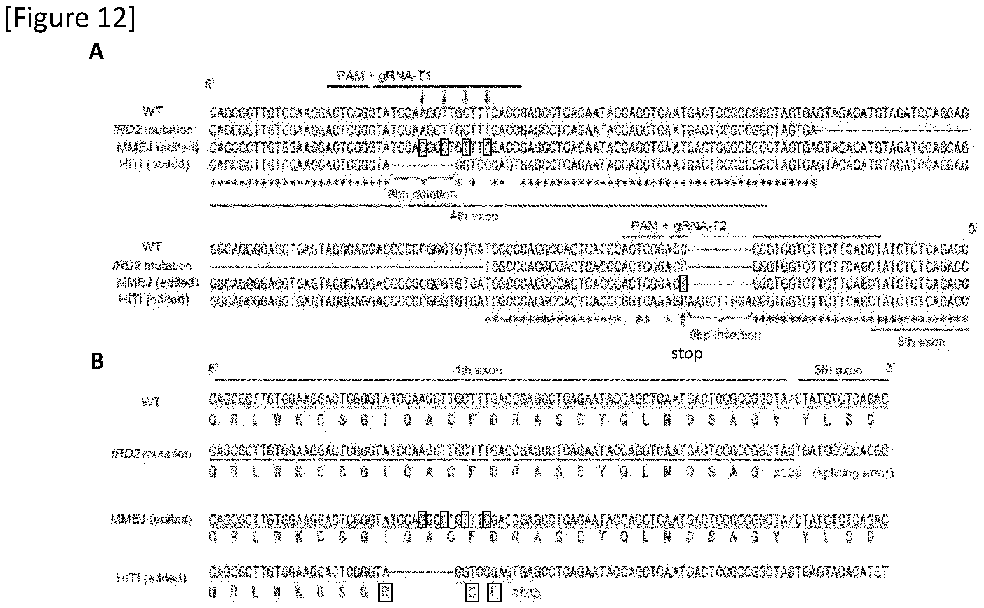

[Figure 12] (A) Comparison of results of editing at the genomic level after application of MMEJ-mediated and HITI-mediated mutation replacements. Mutations induced in a gRNA target site are highlighted with an enclosed letters and arrows and nucleotides preserved throughout four sequences are labeled with * below the sequence alignment. (B) Comparison of results of editing at an amino acid level after application of MMEJ-mediated and HITI-mediated mutation replacement. Amino acids changed with respect to a wild-type sequence are highlighted with enclosed letters. After HITI-mediated mutation replacement, nucleotides of a 5' gRNA target site is changed, and after three missense mutations and loss of 9 bases, nonsense mutation occurred at the 4th exon.
[Figure 13] GNAT1 staining. The arrows show GNAT1-positive photoreceptors. Left: section, right: flatmount.
[Figure 14] Co-localization of mK01 probing Sacas9 expression and GNAT1 immuno-positivity (insert). GNAT-positive cell is observed only at a region introduced with mK01. N=4.
[Figure 15] RT-PCR of Rho, Pde6b, Rcvn and Pkcα. Each sample N=4.
[Figure 16] Rescue efficiency by RT-PCR of Gnat1. Comparison with a Pde6c ^{cpfl1/cpfl1} mouse. Each sample N=4.
[Figure 17] (A) 6-Hz flicker electroretinogram. N=9, 9, 4, 4 and 4 with respect to untreated (NoTx), MMEJ, MMEJ+L-AP4, NoMHA and NoTS, respectively, wherein in MMEJ+L-AP4, MMEJ and L-AP4 were successively administered, (B) amplitude (1.0 log.cd.s./m²) and, (C) recovery (rescue) efficiency (%) for responses from a Pde6c ^{cpfl1/cpfl1} mouse are shown.
[Figure 18] In vitro on-target sequence analysis result. Success: successful mutation replacement without introducing unexpected mutation, Cleavage site indel: insertion and/or loss at any gRNA cleavage site without replacement of IRD2 mutation, AAV plasmid integration: unexpected AAV genome incorporation, Deletion: deletion caused by IRD2 mutation, Other indel: other insertion and/or loss.
[Figure 19] In vivo on-target sequence analysis result. Success: successful mutation replacement without introducing unexpected mutation, Cleavage site indel: insertion and/or loss at any gRNA cleavage site without replacement of IRD2 mutation, AAV plasmid integration: unexpected AAV genome incorporation, Deletion: deletion caused by IRD2 mutation, Other indel: other insertion and/or loss.
[Figure 20] T7E1 assay of t sites (total of 14 sites) of gRNA (A) and gRNA4 (B) expected at CRISPOR (http://crispor.tefor.net/). Expected DNA sizes before T7E1 digestion (Uncut) and after T7E1 digestion (Cut) are shown below the representative gel image from four independent repeated experimentation result. It should be noted that there are no bands of the expected sizes when off-target mutation exists.
[Figure 21] Recovery of vision by in vivo mutation replacement genome editing in a Pde6 ^{cpfl1/cpfl1}Gnat1 ^{IRD2/IRD2} mouse. fVEP (flash visually evoked potential) recorded from the visual cortex contralateral to the treated eye against various intensities of flashes, MMEJ: eye treated by Gnat1 mutation replacement (N=9), OE (over expression): Gnat1 gene supplementation (N=6), all delivered by a single AAV. NoTx: untreated eye (N=9). (A) intensity of stimulation, (B) P1-N1 amplitude, (C) N1-P2 amplitude, (D) threshold. *P<0.05.
[Figure 22] (A) mouse fear-conditioning test, (B) a graph showing freezing time before (Baseline) and during (Stimulus) exposure to a fear-conditioned light cue in each group of untreated Pde6 ^{cpfl1/cpfl1} mice, untreated Pde6 ^{cpfl1/cpfl1}Gnat1 ^{IRD2/IRD2} mice and MMEJ vector-administered Pde6 ^{cpfl1/cpfl1}Gnat1 ^{IRD2/IRD2} mice.
[Figure 23] Measurement of visual acuity by quantifying oculomotor reaction. The data shows average value ± standard error. MMEJ, OE and NoTx are N=10, 7 and 4, respectively. Control Pde6 ^{cpfl1/cpfl1} mouse N=6, *P<0.05, ns: no significant difference.

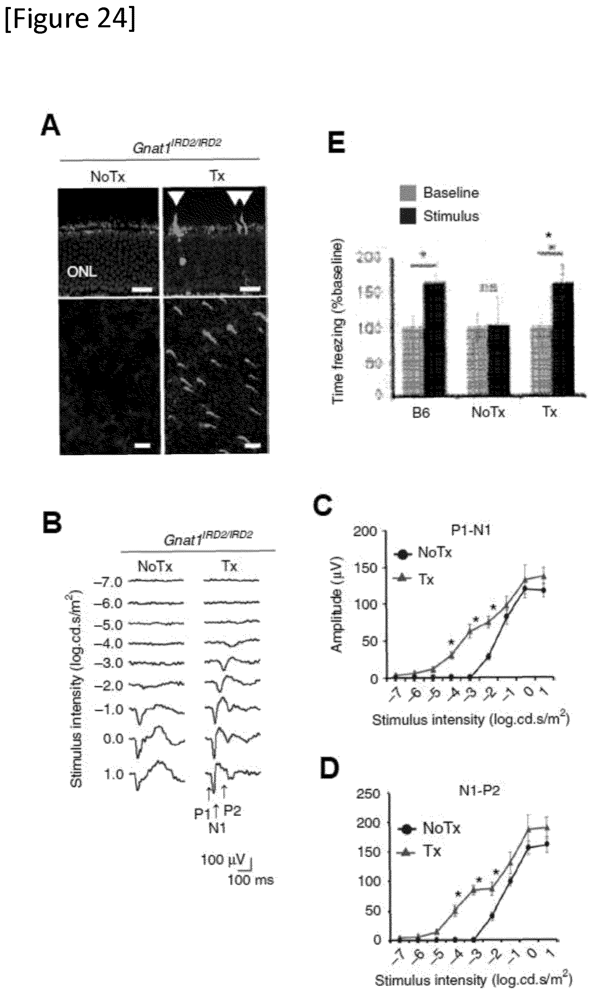

[Figure 24] In vivo mutation replacement genome editing in a retinal degeneration mouse model. (A) Gnat1-positive photoreceptors after treatment of a Gnat1 ^{IRD2/IRD2} mouse (arrowhead). Retina section (top), retinal flatmount (bottom). Scale bar: 20µm. (B) to (D) fVEP from the visual cortex contralateral to the treated eye and non-treated eye of the same mouse. N=7. (B) Intensity of stimulation, (C) P1-N1 amplitude, (D) N1-P2 amplitude. (E) Fear-conditioning test. a graph showing freezing time before (Baseline) and during (Stimulus) exposure to fear-conditioned light cue. Treated (Tx, N=7) and untreated (NoTx, N=6) Gnat1 ^{IRD2/IRD2} mouse, CL57B6 mouse (B6, N=6). The data shows average value ± standard error. ONL outer nuclear layer, *P<0.05, ns: no significant difference.
[Figure 25] Treatment of a Gnat1 ^{IRD2/IRD2} mouse by MMEJ-mediated mutation replacement. (A) Genome editing efficiency measured by RT-PCR (N=5) in a Gnat1 ^{IRD2/IRD2} mouse, (B) and (C) Flicker ERG (N=7), (D) and (E) OKR(N=8), (F) pVEP(N=7). The data shows average value ± standard error. *P<0.05, NoTx: untreated, ns: no significant difference.
[Figure 26] Design of donor sequence and AAV vector.
[Figure 27] In vitro on-target sequence analysis result. Success: successful mutation replacement without introducing unexpected mutation, Cleavage site indel: insertion and/or loss at any gRNA cleavage site without replacement of IRD2 mutation, AAV plasmid integration: unexpected AAV genome incorporation, Deletion: deletion caused by IRD2 mutation, Other indel: other insertion and/or loss.

### [Description of Embodiments]

The embodiments of the present invention are explained below while referring to the drawings.

According to the first aspect of the present invention, an adeno-associated virus (AAV) vector for inserting a desired nucleic acid into a nucleic acid genome in a cell is provided. A genome refers to genetic information of an organism. A nucleic acid genome refers to a genome that is a nucleic acid (especially DNA or RNA) and is interchangeably used with "genomic nucleic acid" herein.

The subject whose cell is transfected with the AAV vector of the first aspect of the present invention includes human; non-human mammals such as cow, pig, sheep, goat, rabbit, dog, cat, guinea pig, hamster, mouse, rat and monkey; birds; fish such as zebrafish; amphibians such as frog; reptiles; insects such as Drosophila; crustaceans, and the like. Preferably, the subject is a human and a non-human mammal.

In addition, the cell that becomes a host cell may be a cell in an organism, an isolated primary cell, or a cultured cell. Since a genome is an element common to all cells, a developed genome editing technique can be applied to a cell of nervous system tissue or cells of all organs other than nervous system tissue. For example, the cell can be, but not limited to, a photoreceptor, retinal pigment epithelial cell, retinal bipolar cell, retinal ganglion cell, retinal horizontal cell, retinal astrocyte, retinal Mueller cell, amacrine cell, retinal vascular endothelial cell, corneal endothelial cell, corneal epithelial cell, keratocyte, iris epithelial cell, ciliary epithelial cell, trabecular cell, or the like. The cell is preferably a retinal cell such as a photoreceptor in the point that cancer is less likely to develop. However, a cell of the same central nervous system, which is a cell of a brain or spinal cord (cone cell, stellate cell, granule cell, Purkinje cell, microglia, oligodendrocyte, astrocyte, or the like) is also a subject.

While the cell can be a cell of a normal subject, the cell is preferably a cell of a subject having a disease, and more preferably a cell of a target having a hereditary disease. Herein, a hereditary disease refers to a disease wherein one factor causing the disease is mutated in a gene.

Examples of the disease or hereditary disease mainly includes ocular diseases such as hereditary retinal degeneration, glaucoma, cataract, uveitis, optic neuritis, diabetic retinopathy, retinal vascular occlusion disease, age-related macular degeneration, corneal dystrophy, bullous keratopathy and corneal opacity, and also includes, but not limited to, at least one selected from the group consisting of Parkinson's disease, Huntington's disease, ocular disease (e.g., macular degeneration, retinal degeneration), Alzheimer's disease, multiple sclerosis, rheumatoid arthritis, Crohn's disease, Peyronie's disease, ulcerative colitis, cerebral ischemia (stroke), myocardial infarction (heart attack), brain injury and/or spinal cord injury, reperfusion injury, ALS, Down syndrome, cataract, schizophrenia, epilepsy, human leukemia and other cancers, and diabetes.

The nucleic acid in the cell to which the AAV vector is inserted includes a nucleic acid including a region consisting of a first nucleotide sequence, a target nucleic acid sequence and a region consisting of a second nucleotide sequence in order in a direction from the 5' end to the 3' end.

The nucleic acid in the cell further has a first gRNA target sequence recognized by a first gRNA of the AAV vector and a first PAM sequence between the region consisting of the first nucleotide sequence and the target nucleic acid sequence and has a second gRNA target sequence recognized by a second gRNA of the AAV vector and a second PAM sequence between the target nucleic acid sequence and the region consisting of the second nucleotide sequence. The first gRNA target sequence and the first PAM sequence may be in either the sense strand or the antisense strand of the AAV vector, and the second gRNA target sequence and the second PAM sequence may be in either the sense strand or the antisense strand of the AAV vector. The first gRNA target sequence and the second gRNA target sequence may target either sense strand or the antisense strand of the nucleic acid in the cell.

The first gRNA target sequence of the nucleic acid in the cell recognized by the first gRNA of the AAV vector and the second gRNA target sequence of the nucleic acid in the cell recognized by the second gRNA of the AAV vector may be difference sequences, or may be the same sequence.

In one embodiment, the nucleic acid in the cell has a first gRNA target sequence and a first PAM sequence between the region consisting of the first nucleotide sequence and the target nucleic acid sequence and has a second gRNA target sequence and a second PAM sequence between the target nucleic acid sequence and the region consisting of the second nucleotide sequence in order in a direction from the 5' end to the 3' end. The first gRNA target sequence and the second gRNA target sequence are sequences recognized by the first gRNA and the second gRNA of the AAV vector, respectively. In the case of this embodiment, the first gRNA and the second gRNA of the AAV vector both target the sense strand of the nucleic acid in the cell.

In another embodiment, the nucleic acid in the cell has a first gRNA target sequence and a first PAM sequence between the region consisting of the first nucleotide sequence and the target nucleic acid sequence and has a complementary sequence of a second PAM sequence and a complementary sequence of a second gRNA target sequence between the target nucleic acid sequence and the region consisting of the second nucleotide sequence in order in a direction from the 5' end to the 3' end. In the case of this embodiment, the first gRNA of the AAV vector targets the sense strand of the nucleic acid of the cell and the second gRNA of the AAV vector targets the antisense strand of the nucleic acid in the cell.

In another embodiment, the nucleic acid in the cell has a complementary sequence of a first PAM sequence and a complementary sequence of a first gRNA target sequence between the region consisting of the first nucleotide sequence and the target nucleic acid sequence and has a second gRNA target sequence and a second PAM sequence between the target nucleic acid sequence and the region consisting of the second nucleotide sequence in order in a direction from the 5' end to the 3'. In the case of this embodiment, the first gRNA of the AAV vector targets the antisense strand of the nucleic acid in the cell and the second gRNA of the AAV vector targets the sense strand of the nucleic acid in the cell.

In another embodiment, the nucleic acid in the cell has a complementary sequence of a first PAM sequence and a complementary sequence of a first gRNA target sequence between the region consisting of the first nucleotide sequence and the target nucleic acid sequence and has a complementary sequence of a second PAM sequence and a complementary sequence of a second gRNA sequence between the target nucleic acid sequence and the region consisting of the second nucleotide sequence in order in a direction from the 5' end to the 3'. In the case of this embodiment, the first gRNA of the AAV vector and the second gRNA of the AAV vector both target the antisense strand of the nucleic acid in the cell.

The target nucleic acid sequence is a nucleic acid sequence sought to be replaced using the AAV vector of the first aspect of the present invention, and is preferably a part of a gene. The target nucleic acid sequence preferably has one or more (e.g., 2, 3, 4, or 5 or more) mutation type base, and more preferably has one or more (e.g., 2, 3, 4, or 5 or more) mutation type base associated with a genetic disease. Mutation includes replacement, deletion, and/or addition. It is well known that mutation of one or more base may be a cause of a disease, and replacement of one or more mutation type base in the genomic nucleic acid in the cell with a normal type base using a below-discussed AAV vector can correct the mutation of DNA of a cell in a subject and, consequently, can contribute to therapy of a disease. However, a gene region or a promoter region not having mutation may be a subject for genome editing.

The length of the target nucleic acid sequence is not particularly limited, but can be 1 to 1500 in base length, preferably 1 to 700 in base length, and more preferably 1 to 500 in base length.

The sequences consisting of nucleotides of 17 to 24 in base length on the 5' end side of the PAM sequences (first PAM sequence and second PAM sequence) of the upstream and downstream of the target nucleic acid sequence are first gRNA target sequence and second gRNA target sequence, respectively. As discussed below, the AAV vector of the first aspect of the present invention is configured to have a sequence encoding the first gRNA recognizing the first gRNA target sequence and a sequence encoding the second gRNA recognizing the second gRNA target sequence.

The first gRNA target sequence and the first PAM sequence are preferably adjacent. In addition, the second gRNA target sequence and the second PAM sequence are preferably adjacent.

The PAM (protospacer adjacent motif) sequence is a sequence that is well known in the art, which is embedded in nucleic acids in a cell. Therefore, the PAM sequence in the nucleic acid in the cell differs depending on the type of Cas9. For example, in saCas9 derived from Staphylococcus aureus, the PAM sequence includes 5'-NNGRRT-3' (R is A or G, N is any nucleotide), and the length is generally 3 to 8 in base length.

The above-described first nucleotide sequence and the above-described second nucleotide sequence act as a homology arm for causing a microhomology-mediated joining (Microhomology-mediated end-joining, MMEJ) of the genomic nucleic acid in the cell and the nucleic acid sequence from the vector.

The region consisting of the first nucleotide sequence consists of a nucleotide sequence which is preferably 5 to 40 in base length, more preferably 10 to 30 in base length, and even more preferably 12 to 20 in base length.

The region consisting of the second nucleotide sequence consists of a nucleotide sequence which is preferably 5 to 40 in base length, more preferably 10 to 30 in base length, and even more preferably 12 to 20 in base length.

The AAV vector of the first aspect of the present invention includes the property of expressing a Cas9 nuclease with one vector, the property of expressing gRNA, the property of cleaving a nucleic acid region including a target nucleic acid sequence in a genomic nucleic acid in a cell, the property of cutting out a nucleic acid fragment including a desired nucleic acid from the vector itself and the property of inserting the desired nucleic acid into a nucleic acid in a cell, wherein a desired nucleic acid can accurately and readily be inserted.

The AAV vector includes inverted terminal repeat (ITR) necessary for efficient proliferation of AAV genome at both ends of a DNA strand.

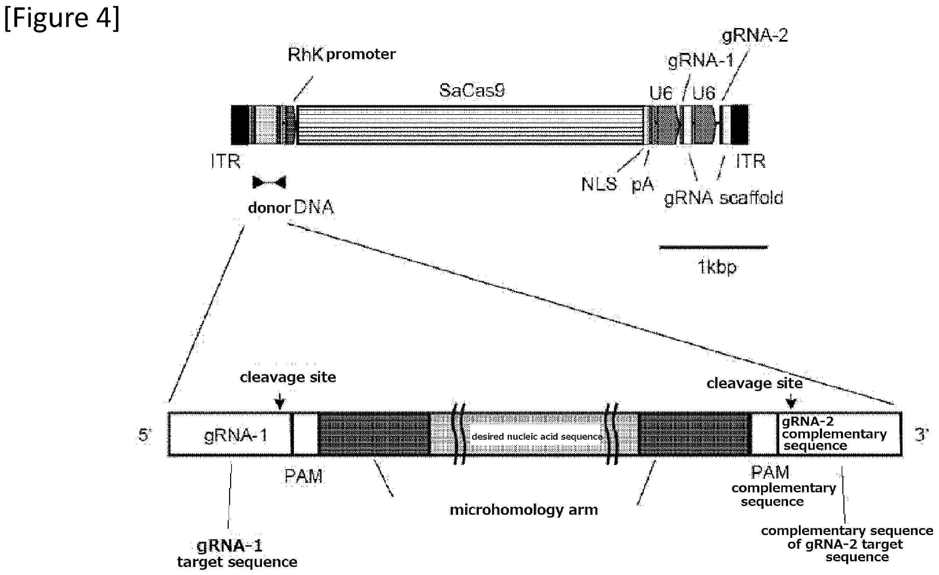

As shown in Figure 4, in one preferable embodiment, the AAV vector includes a first gRNA target sequence (the first gRNA targets a sense strand), a first PAM sequence, a region consisting of a first nucleotide sequence (microhomology arm), a desired nucleic acid, a region consisting of a second nucleotide sequence (microhomology arm), a second PAM sequence (which is a complementary sequence since an antisense strand is targeted), a second gRNA target sequence (the second gRNA is a complementary sequence since an antisense strand is targeted), a cell-specific promoter (RhK promoter in the drawing), a sequence encoding a Cas9 nuclease (SaCas9 in the drawing), a first RNA polymerase III promoter (U6 in the drawing), a sequence encoding a first gRNA recognizing a first gRNA target sequence (gRNA-1 in the drawing), a Scaffold sequence, a second RNA polymerase III promoter (U6 in the drawing), a sequence encoding a second gRNA recognizing a second gRNA target sequence (gRNA-2 in the drawing) and a Scaffold sequence.

The first gRNA target sequence and the second gRNA target sequence of the AAV vector may be different sequences, or may be the same sequence.

The first gRNA and the second gRNA of the AAV vector may target the first gRNA target sequence and the second gRNA target sequence in either the sense strand or the antisense strand of the AAV vector.

Thus, for example, instead of the AAV vector of Figure 4, an AAV vector may have a first PAM sequence and a first gRNA target sequence in the antisense strand such that the first gRNA targets the antisense strand. In addition, instead of the AAV vector of Figure 4, an AAV vector may have a second gRNA target sequence and a second PAM sequence in the sense strand such that the second gRNA targets the sense strand.

The AAV vector preferably has the above-described elements in order from the 5' end to the 3' end, but the order of the arrangement of the elements is not limited as long as the effect of the present invention is achieved.

In other words, the order may be changed regarding the following four cassettes: (1) an expression cassette consisting of a region including a first gRNA target sequence, a first PAM sequence, a region consisting of a first nucleotide sequence, a desired nucleic acid, a region consisting of a second nucleotide sequence, a second gRNA target sequence and a second PAM sequence (including both the case in which the first gRNA target sequence and first PAM sequence are in the sense strand or the antisense strand and the case in which the second gRNA target sequence and second PAM sequence are in the sense strand or the antisense strand); (2) a Cas9 expression cassette consisting of a region including a cell-specific promoter and a sequence encoding a Cas9 nuclease; (3) a first gRNA expression cassette consisting of a region including a first RNA polymerase III promoter, a sequence encoding a first gRNA recognizing a first gRNA target sequence and a Scaffold sequence; and (4) a second gRNA expression cassette consisting of a region including a second RNA polymerase III promoter, a sequence encoding a second gRNA recognizing a second gRNA target sequence and a Scaffold sequence. For example, the positions of (3) and (4) may be switched.

Furthermore, (3) the first gRNA expression cassette and (4) the second gRNA expression cassette can be one gRNA expression cassette. In such a case, there will be an RNA polymerase III promoter, a sequence encoding a first gRNA, a Scaffold sequence, a sequence encoding a second gRNA and a Scaffold sequence. The sequence encoding the first gRNA and the sequence encoding the second gRNA are switchable.

The first gRNA target sequence and the first PAM sequence of the AAV vector are preferably adjacent. The first gRNA target sequence is preferably a sequence consisting of a nucleotide which is 17 to 24 in base length. The first PAM sequence is preferably a sequence consisting of a nucleotide which is 3 to 8 in base length. Preferably, the first gRNA target sequence and the first PAM sequence include a sequence consisting of a nucleotide which is 17 to 24 in base length and complementary to the first gRNA sequence and a first PAM sequence which is 3 to 8 in base length and adjacent thereto. The base length of the entirety of the first gRNA target sequence and the first PAM sequence in the AAV vector is preferably 20 to 32, and more preferably 26 to 30.

The first PAM sequence includes, for example, but not limited to, NRG (R is A or G), NGA, NNAGAAW (W is A or T), NNNNGMTT (M is A or C), NNGRRT (R is A or G) and the like.

The second gRNA target sequence and the second PAM sequence of the AAV vector are preferably adjacent. The second gRNA target sequence is preferably a sequence consisting of a nucleotide which is 17 to 24 in base length. The second PAM sequence is preferably a sequence consisting of a nucleotide which is 3 to 8 in base length. Preferably, the second gRNA sequence and the second PAM sequence include a sequence consisting of a nucleotide which is 17 to 24 in base length and complementary to the second gRNA sequence and a second PAM sequence which is 3 to 8 in base length and adjacent thereto. The base length of the entirety of the second gRNA target sequence and the second PAM sequence in the AAV vector is preferably 20 to 32, and more preferably 26 to 30.

The second PAM sequence includes, for example, but not limited to, NRG (R is A or G), NGA, NNAGAAW (W is A or T), NNNNGMTT (M is A or C), NNGRRT (R is A or G) and the like.

The first gRNA target sequence of the AAV vector may be any sequence as long as the sequence is recognized by the above-described first gRNA expressed by the vector. Preferably, the first gRNA target sequence of the AAV vector is the same sequence as the above-described first gRNA target sequence of the nucleic acid in the cell.

The second gRNA target sequence of the AAV vector may be any sequence as long as the sequence is recognized by the above-described second gRNA expressed by the vector. Preferably, the sequence is the same as the above-described second gRNA target sequence of the nucleic acid in the cell.

The order of the first gRNA target sequence of the AAV vector and the second gRNA target sequence of the AAV vector may be switched. In addition, the first gRNA target sequence and the second gRNA target sequence of the AAV vector may be different sequences, or may be the same sequence.

The first and second PAM sequences of the AAV vector are preferably the same sequences as the above-described first and second PAM sequences of the nucleic acid in the cell.

In order to ensure that the region consisting of the first nucleotide sequence, the desired nucleic acid and the region consisting of the second nucleotide sequence of the AAV vector are cleaved by the below-discussed Cas9 nuclease as one continuous fragment, in other words, in order to avoid the sequence of the AAV vector being cleaved by the Cas9 nuclease at a sequence between the region consisting of the first nucleotide sequence and the desired nucleic acid and a sequence between the desired nucleic acid and the region consisting of the second nucleotide sequence, it is preferable that those sequences are mutated from a natural (wild type or native) sequence as needed.

It is preferable to have a configuration which do not have the same sequence as the above-described first gRNA target sequence, the same sequence as the above-described second gRNA target sequence, or both of the AAV vector between the region consisting of the first nucleotide sequence and the region consisting of the second nucleotide sequence. More specifically, it is preferable that the AAV vector does not have the same sequence as the above-described first gRNA target sequence, the same sequence as the above-described second gRNA target sequence, or both in both the sense strand and the antisense strand between the region consisting of the first nucleotide sequence and the region consisting of the second nucleotide sequence.

In one embodiment, when the same sequence as the first gRNA target sequence exists between the region consisting of the first nucleotide sequence and the region consisting of the second nucleotide sequence, more specifically, between the region consisting of the first nucleotide sequence and the desired nucleic acid, the first gRNA target sequence can be mutated. In another embodiment, when the same sequence as the second gRNA target sequence exists between the region consisting of the first nucleotide sequence and the region consisting of the second nucleotide sequence, more specifically, between the desired nucleic acid and the region consisting of the second nucleotide sequence, the second gRNA target sequence can be mutated. In yet another embodiment, when both the same sequence as the first gRNA target sequence and the same sequence as the second gRNA target sequence exist between the region consisting of the first nucleotide sequence and the region consisting of the second nucleotide sequence, both the first gRNA target sequence and the second gRNA target sequence can be mutated. By employing such a configuration, it is possible to avoid cleavage of the sequence of the AAV vector by the Cas9 nuclease in the sequence between the region consisting of the first nucleotide sequence and the desired nucleic acid and/or the sequence between the desired nucleic acid and the region consisting of the second nucleotide sequence.

In one embodiment, when the same sequence as first gRNA target sequence exists between the region consisting of the first nucleotide sequence and the region consisting of the second nucleotide sequence, more specifically, between the region consisting of the first nucleotide sequence and the desired nucleic acid, it is possible to mutate the first PAM sequence adjacent to the first gRNA target sequence on the side close to the region consisting of the second nucleotide sequence. In another embodiment, when the same sequence as the second gRNA target sequence exists between the region consisting of the first nucleotide sequence and the region consisting of the second nucleotide sequence, more specifically, between the desired nucleic acid and the region consisting of the second nucleotide sequence, it is possible to mutate the second PAM sequence adjacent to the second gRNA target sequence on the side close to the region consisting of the first nucleotide. In yet another embodiment, when both the same sequence as the first gRNA target sequence and the same sequence as the second gRNA target sequence exist between the region consisting of the first nucleotide sequence and the region consisting of the second nucleotide sequence, it is possible to mutate both the first PAM sequence adjacent to the first gRNA target sequence on the side close to the region consisting of the second nucleotide sequence and the second PAM sequence adjacent to the second gRNA target sequence on the side close to the region consisting of the first nucleotide sequence. By employing such a configuration, it is possible to avoid cleavage of the sequence of the AAV vector by the Cas9 nuclease in the sequence between the region consisting of the first nucleotide sequence and the desired nucleic acid and/or the sequence between the desired nucleic acid and region consisting of the second nucleotide sequence.

Mutation of a nucleotide sequence within the AAV vector can be performed using the well-known technique in the subject technical field.

The Cas9 nuclease is an enzyme that has two DNA cleavage domains (HNH domain and RuvC domain), forms a complex with a guide RNA (gRNA), and cleaves the target part of the nucleic acid.

The Cas9 nuclease is not particularly limited, and includes any wild-type Cas9 nuclease and a mutant thereof having nuclease activity. Such a mutant includes a nuclease that underwent replacement, loss and/or addition of 1, 2, 3, 4, 5, 6, 7, 8, 9, 10, 15, 20, 25, 30, 40, 45, or 50 amino acids of the wild-type Cas9 nuclease. Examples of Cas9 nuclease include SpCas9 derived from Streptococcus pyrogenes, StCas9 derived from Streptococcus thermophilus, SaCas9 derived from Staphylococcus aureus (Nature 520: 186-191, 2015) and the like. SaCas9 is preferable in terms of having a small size and being able to increase free space in the AAV vector.

Targeting of a nucleic acid mediated by a sequence of at least 17 to 20 nucleotides in the 5' end of gRNA (the first gRNA and the second gRNA). Such a nucleotide is designed to be complementary to the complementary strand (opposite strand) of the nucleic acid sequence (the first gRNA target sequence and the second gRNA target sequence) adjacent to the PAM sequence of the nucleic acid. The targeting may occur by interaction by base pair complementarity between the complementary strand (opposite strand) of the nucleic acid sequence (the gRNA target sequence and the second gRNA target sequence) and gRNA (the first gRNA and the second gRNA) through the above-described nucleotides of at least 17 to 20 bases. Therefore, specific double-strand cleavage is carried out to both the vector and the nucleic acid including the target nucleic acid sequence in the cell using the Cas9 nuclease.

The portion where double-strand cleavage occurs is often positioned 3 bases upstream of the PAM sequence (5' end side) but also may be positioned at a different site in the gRNA target sequence. The gRNA recognizes a base sequence positioned on the 5' side of the PAM. Therefore, the Cas9 nuclease carries out specific cleavage to the first gRNA target sequence and the second gRNA target sequence of the nucleic acid in the cell and carries out specific cleavage to the first gRNA target sequence and the second gRNA target sequence of the AAV vector. Upon coupling the nucleic acid fragment generated from the vector and the nucleic acid in the cell, the remaining nucleic acid sequence (mostly 3 bases) from the PAM and its upstream gRNA target sequence is lost. Thus, the coupled nucleic acid will not undergo another cleavage by a nuclease existing in the cell and will be stably retained.

The first gRNA of the vector forms a complex with the Cas nuclease to recognize the first gRNA target sequence of the nucleic acid in the cell and the second gRNA of the vector forms a complex with the Cas nuclease to recognize the second gRNA target sequence of the nucleic acid in the cell, wherein the double strand cleavage cuts out a nucleic acid fragment including a region consisting of the sequence downstream of the site cleaved by the Cas9 nuclease of the first gRNA target sequence of the nucleic acid in the cell, the first PAM sequence, the target nucleic acid sequence and the sequence upstream of the site cleaved by the Cas9 nuclease in the second gRNA target sequence, and the region consisting of the first nucleotide sequence and the region consisting of the second nucleotide sequence remain as a nucleic acid in the cell without being cut out.

The first gRNA of the vector forms a complex with the Cas nuclease to recognize the first gRNA target sequence of the vector and the second gRNA of the vector forms a complex with the Cas nuclease to form the second gRNA of the vector, wherein the double strand cleavage generates a nucleic acid fragment including the region consisting of the first nucleotide sequence, the desired nucleic acid and the region consisting of the second nucleotide sequence from the vector.

The above-described desired nucleic acid of the vector is a nucleic acid intended to replace the above-described target nucleic acid sequence of the nucleic acid in the cell, and may be any nucleic acid of which introduction to the nucleic acid in the cell is desired. Preferably, the desired nucleic acid is a nucleic acid having a sequence identical to the above-described target nucleic acid sequence other than the fact that the position of the mutation type base of the above-described target nucleic acid sequence of the nucleic acid in the cell is a normal type base. More preferably, the desired nucleic acid is a nucleic acid having a sequence identical to the above-described target nucleic acid sequence other than the fact that the position of the mutation type base of the target sequence which is a gene of the cell is a normal type base.

The length of the desired nucleic acid can be 1 to 1500 in base length, preferably it can be 1 to 700 in base length, and more preferably 1 to 500 in base length.

The first nucleotide sequence included in the nucleic acid in the cell and the first nucleotide sequence included in the vector preferably have an identical sequence. In addition, the second nucleotide sequence included in the nucleic acid in the cell and the second nucleotide sequence included in the vector preferably have an identical sequence. Therefore, the first nucleotide sequence in the nucleic acid in the cell and the first nucleotide sequence in the vector are linked by microhomology-mediated joining (microhomology-mediated end-joining, MMEJ) and the second nucleotide in the nucleic acid in the cell and the second nucleotide sequence in the vector are linked by a microhomology-mediated joining, whereby the desired nucleic acid is inserted in the nucleic acid at a position between the first nucleotide sequence and the second nucleotide sequence in the nucleic acid in the cell.

Therefore, the AAV vector of the first aspect can also be referred to as a vector for replacing the target nucleic acid of the nucleic acid in the cell with the desired nucleic acid of the vector. The microhomology-mediated joining is a mechanism discovered as a DNA repair mechanism that eukaryote has, which is a mechanism in which complementary sequences binds to each other between both ends that are about 5 to 25 in base length caused upon double strand cleavage of DNA to repair the DNA (see NATURE PROTOCOL, Vol. 11, No. 1 published online on line 17 December 2015, doi: 10.1038 / nprot.2015.140) .

A cell-specific promoter can be appropriately selected by those with skilled techniques, according to the type of the cell. The cell-specific promoter can be a natural promoter or a part thereof, or can be a synthetic promoter.

Such a cell-specific promoter includes a natural cell-specific promoter, a promoter having 50 to 150 continuing base sequences of those promoters, or a promoter consisting of a base sequence that is 90% or more identical with respect to the 50 to 150 continuing base sequences of those promoters.

A natural cell-specific promoter used for a retina includes, but not limited to, rhodopsin kinase promoter, RPE65 promoter, Best1 promoter, mGluR6 promoter, cone arrestin promoter, CRALBP1 promoter, Chx10 promoter, rhodopsin promoter, cone opsin promoter, recoverin promoter and the like. In addition, a promoter for other organs includes, but not limited to, a promoter selected from synapsin I promoter, myelin basic protein promoter, neuron-specific enolase promoter, calcium/calmodulin-dependent protein kinase II promoter, tubulin α I promoter, platelet-derived growth factor β strand promoter, glial fibrillary acidic protein (GFAP), L7 promoter and glutamate receptor delta 2 promoter.

The length of the cell-specific promoter is not particularly limited, but is preferably short in terms of increasing free space of the vector, preferably 500 bases or less in base length, more preferably 200 bases or less, more preferably 150 bases or less, more preferably 120 bases or less, and even more preferably 100 bases or less. By using a cell-specific promoter that is short in base length, an introduction fragment with a length sufficient for knock-in of a gene using MMEJ can be loadable to a single vector of an AAV in accordance with the disease.

When preparing a synthetic promoter based on the sequence of a natural promoter by replacement, deletion, or addition of a portion thereof, for example, those skilled in the art can use their normal technique to prepare a synthetic promoter with shortened base length while maintaining the function of the cell-specific promoter by adopting a region with high preservation between species.

An RNA polymerase III promoter is a promoter that enables expression of gRNA in a mammalian cell after transfection of the vector. An RNA polymerase III promoter includes a U6 promoter, H1 promoter, 7SK promoter and the like, wherein a U6 promoter is preferred in terms of driving a relatively short base sequence.

The sequence encoding the first gRNA that recognizes the first gRNA target sequence is preferably 17 to 24 in base length.

The first gRNA is driven by the U6 promoter, forms a complex with Cas and recognizes and cleaves the first gRNA target sequence.

The sequence encoding the second gRNA that recognizes the second gRNA target sequence is preferably 17 to 24 in base length.

The second gRNA is driven by the U6 promoter, forms a complex with Cas and recognizes and cleaves the second gRNA target sequence.

The above-described Scaffold sequence is a sequence encoding tracrRNA and supports the binding of a Cas9 nuclease to a target DNA. The sequences that configure such a site is known.

The vector can include a nuclear localization sequence (NLS) that causes nuclear transfer of a protein, wherein a known and suitable NLS can be used. For example, many NLSs have a plurality of basic amino acids called bipartite basic repeats (Biochim.Biophys.Acta, 1991, 1071: 83-101). NLS including bipartite basic repeats can be placed at any portion in the nucleic acid sequence, causing nuclear localization of the expressed protein.

The vector can further include any signal necessary for efficient polyadenylation of the transcript, transcription termination, ribosome binding site, or translation termination. Such sites are well known in the subject technical field and those skilled in the art would be able to select a suitable sequence.

While the above-described AAV vector of the first aspect of the present invention is immediately applicable to many mutation correction therapies targeting retinal photoreceptors, the AAV vector can be highly versatile, applicable for therapy of diseases other than ocular diseases by using a small promoter that can be used in other tissues.

According to the second aspect of the present invention, a kit for inserting a desired nucleic acid into a nucleic acid in a cell, the kit comprising the above-described vector of the first aspect, is provided.

According to the third aspect of the present invention, a method of inserting a desired nucleic acid in a cell, the method comprising the step of introducing the above-described vector of the first aspect to a cell, is provided.

According to the fourth embodiment of the present invention, a method of manufacturing a cell comprising a desired nucleic acid, the method comprising the step of introducing the above-described vector of the first aspect to a cell and the step of inserting a desired nucleic acid into a nucleic acid in the cell, is provided.

A cell containing desired nucleic acid can be obtained by selecting a cell based on an indicator reflecting insertion of the desired nucleic acid. For example, when the nucleic acid to be inserted includes a gene encoding a specific reporter protein, expression of the reporter protein can be detected to select a cell easily and with high sensitivity using the level of the detected expression as an indicator.

According to the fifth aspect of the present invention, a method for treating a disease, comprising the step of introducing the above-described vector of the first aspect to a cell and the step of inserting a desired nucleic acid into the nucleic acid in the cell, is provided. Such a therapeutic method may be a therapeutic method in a human or a therapeutic method in a non-human, and may be an in vivo therapeutic method or an in vitro therapeutic method. The disease is outlined in the first aspect.

According to the sixth aspect of the present invention, a therapeutic composition for therapy of a disease comprising the above-described vector of the first aspect is provided. The subject of the therapy includes human; non-human mammals such as cow, pig, sheep, goat, rabbit, dog, cat, guinea pig, hamster, mouse, rat and monkey; birds; fish such as zebrafish; amphibians such as frog; reptiles; insects such as Drosophila; crustaceans, and the like. The disease is outlined in the first aspect.

Disclosure of all the patent applications and documents cited herein are hereby incorporated by reference in their entirety.

Hereafter, the present invention will be described in more detail with reference to Examples, but the present invention is not limited thereto.

### [Examples]

### Test 1

### Example 1 Development of photoreceptor-specific minimal promoter

The present inventors designed various synthetic promoters in order to select a promoter having activity in retinal photoreceptors while maintaining photoreceptor specificity.

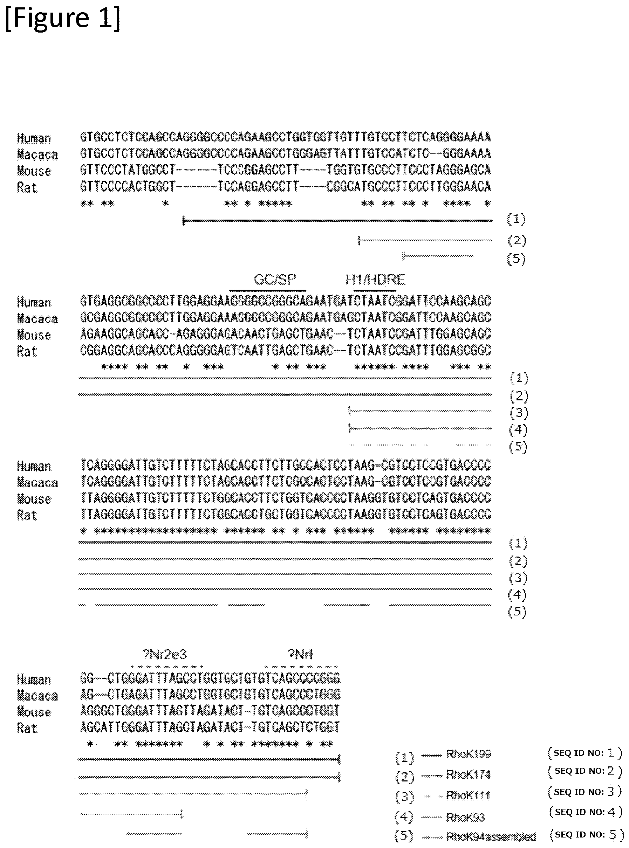

The promoter was designed by modifying a known human Rhodopsin kinase promoter (Khani et al., IOVS 2007; 48: 3954-3961. DOI: 10.1167/iovs.07-0257). As shown in Figure 1, first, multi-alignment analysis was carried out with ClustalW (http://clustalw.ddbj.nig.ac.jp/) using a corresponding region of a cynomolgus monkey (Macaca fascicularisp), mouse (Mus musculus) and rat (Rattus norvegicus) with respect to a known promoter region 199bp (-112/+87). Based on the analysis result, from a region with high preservation between species, from the highly conserved region between species, a sequence of 174bp (-87/+87) (SEQ ID NO: 2), a sequence of 111bp (-29/+82) (SEQ ID NO: 3), and a sequence of 93bp (-29/+64) (SEQ ID NO: 4) were selected. In addition, a sequence of 94bp (SEQ ID NO: 5) was also selected by considering and combining sequences with high preservation and a transcription factor binding domain.

### Example 2 Reporter assay

### (Material and method)

A reporter AAV was made using each type of promoter synthesized based on the sequences selected in Example 1. EGFP (Clontech) which is a green fluorescent protein was placed downstream of the synthetic promoter sequence and incorporated into a multi-cloning site of an AAV shuttle vector plasmid (pAAV-MCS; Cell Biolabs, Inc.). EGFP is driven by a synthetic promoter as a reporter gene. AAV (2/8 capsid) was made by transfecting these shuttle vector plasmids into an HEK293T cultured cell together with a plasmid having a helper gene and a rep/cap gene. Purification of the AAV was carried out with an AKTA prime liquid chromatography system (GE Healthcare) connected to an AVB sepharose column. Titer measurement calculated the number of genome copies of the AAV using a qPCR method. Each reporter AAV that was made was subretinally injected to a mouse (4 × 10⁹ genome copies/eye) and the eye was taken out one week later to histologically assess the expression of an EGFP protein.

### (Result)

As shown in Figure 3A, in the AAV vector, inverted terminal repeat (ITR), each type of promoter, EGFP, a human growth hormone poly A signal (hGh pA) and inverted terminal repeat (ITR) were placed in a direction from the 5' end to the 3' end in the AAV vector. A 331bp CMV-s promoter is a shortened version of the CMV promoter which is a constitutive promoter and is used as a positive control.

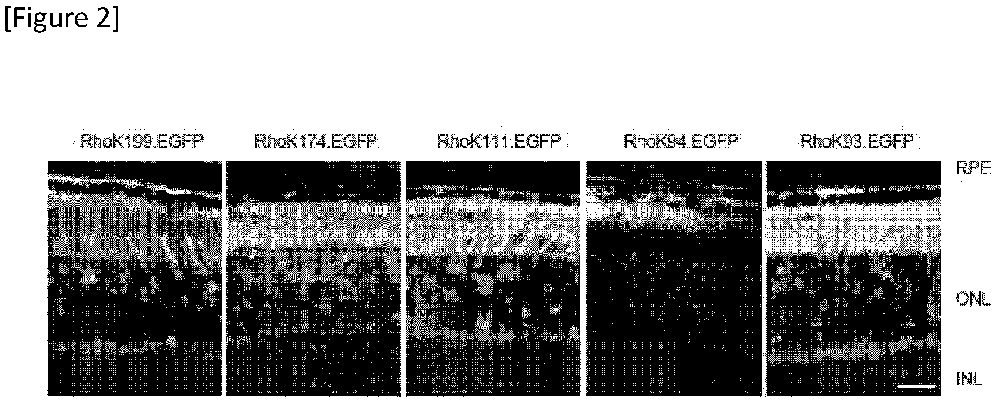

As shown in Figure 2, when gene introduction is carried out with an AAV vector incorporating promoters of the sequences of 199bp, 174bp, 111bp and 93bp, photoreceptor cell-specific expression of EGFP protein was found in all cases (Figure 2). Among the above, the sequence of 93bp was observed to have unexpectedly strong fluorescence in the retina despite being very small, showing that even retinal photoreceptor cell has activity while maintaining cell-specificity of the promoter. Expression of an EGFP protein was not observed in the case of the promoter sequence of 94 bp.

In addition, as shown in Figure 3B, when using RhoK174 and PhoK93, expression of an EGFP reporter protein was found in the photoreceptor cell layer. When using a CMV-s promoter which is a positive contrast, EGFP expression was found in both the photoreceptor cell layer and the retinal pigment epithelial layer. As shown in Figure 3C, Western blot also detected expression of EGFP in the retinal pigment epithelial layer only when using the CMV-s promoter.

### Example 3 Design of All-in-one AAV vector

Assuming to use saCas9 (3.2 kb), a gRNA pair and a donor DNA were designed to treat an IRD mouse (Miyamoto et al., Exp Eye Res. 2010 Jan; 90 (1): 63-9) with inherited retinal degeneration due to a point mutation in Gnat1 gene (Figure 4). The gRNA was designed with RGEN Tools (http://www.rgenome.net/). The donor DNA sequence comprised microhomology arm sequence (20bp) (SEQ ID NOs: 7 and 8) at each of both ends of an insert (146bp in the 4th intron of the Gnat1 gene) (SEQ ID NO: 6) which was further flanked by a gRNA target sequence (gRNA: 21bp + PAM: 6bp) (SEQ ID NOs: 9 and 10) for cutting out the donor sequence. pX601 plasmid (addgene number 61591) was used for the backbone and SaCas9 to generate All-in-one AAV vector. The CMV promoter of pX601 was replaced with the RhoK93 promoter, and a donor sequence and a second gRNA expression cassette were added. The bGH polyA sequence was replaced with a shorter polyA sequence.

### Example 4 Gnat1 rescue

### (Material and method)

An AAV (2/8 capsid) was made by transfecting the All-in-one AAV vector plasmid prepared in Example 3 into an HEK293T cultured cells together with a plasmid having a rep/cap gene and a helper gene. Purification of the AAV was carried out with an AKTA prime liquid chromatography system (GE Healthcare) connected to an AVB sepharose column. Titer measurement calculated the number of genome copies of the AAV using a qPCR method.

Each reporter AAV was subretinally injected to a 6-month-old IRD mouse (4 × 10⁹ genome copies/eye) and the visual evoked potential (VEP) was measured 3 weeks later for assessment. The measurement of VEP was carried out in accordance with the method of Tomiyama and others (Tomiyama et al., PLoS ONE 2016. DOI: 10.1371 / journal.pone.0156927). A stainless screw (0.6 mm in diameter, 4 mm in length) was embedded in the primary visual cortex of the mouse (3.6 mm from bregma to the caudal side, 2.3 mm to the ear side, and 2 mm deep from the skull) under anesthesia one week before the measurement. An LED light stimulator (LS-100), a Ganzfeld dome, and an electric potential recording system (PeREC; all manufactured by Mayo) were used for the measurement. The eye contralateral to the eye assessed was covered with an eye patch, and the difference between the left and right visual cortex was measured.

### (Result)

A Pde6 ^{cpfl1/cpfl1}Gnat1 ^{IRD2/IRD2} mouse is a disease model mouse with defects in Pde6 and Gnat1 which cause functional deficiencies of the rods and the cones and thereby cause blindness. Since only a small cortical response against the strongest light stimulation mediated by Gnat2 remains in this mouse, the therapeutic effect caused by gene mutation can be clearly delineated (Mol. Ther. 26, 2397-2406 (2018); Plos One 5, e15063 (2010).

As shown in Figure **5A**, Gnat1-positive photoreceptors were observed in the photoreceptor layer 3 weeks after AAV injection to a Pde6 ^{cpfl1/cpfl1}Gnat1 ^{IRD2/IRD2} mouse (arrow). As shown in Figure **5B**, an increase in retinal response was not observed one week after AAV-MMEJ-Gnat1 injection (Gnat1-MMEJ). However, as shown in Figure **5C**, a strong response in the mouse appeared 3 weeks after AAV-MMEJ-Gnat1 injection (Gnat1-MMEJ), wherein sensitivity to light of the visual cortex was improved by 10000-fold. As shown in Figure **5D**, rod-photoreceptor phototransduction was recovered by AAV-MMEJ-Gnat1 injection.

### Test 2

### Example 1 Selection of promoter

In this test, a promoter of 93 bp (SEQ ID NO: 4) constructed in Example 1 of Test 1, which is the smallest promoter that maintains retina-specific transcription, was used. In this test, the promoter is called a GRK-1-93 promoter for convenience.

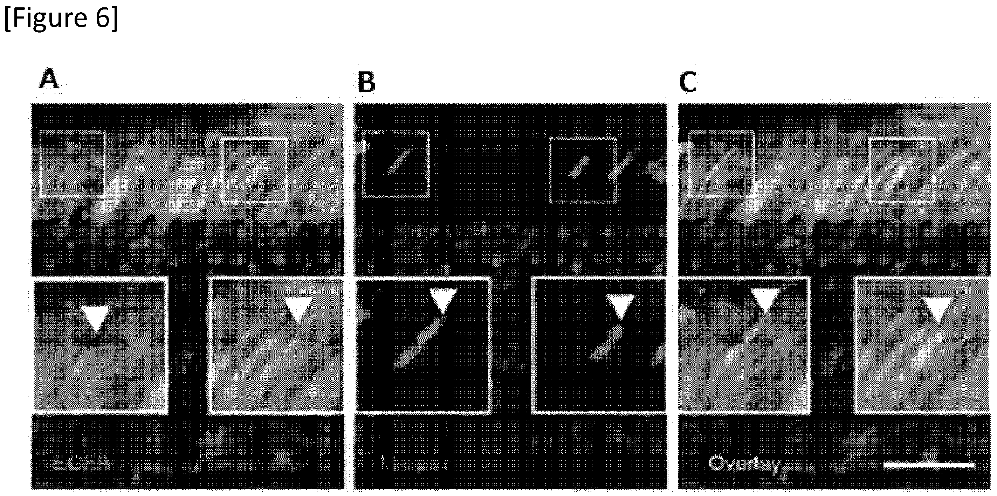

Simultaneous expression of cone-specific M-opsin and EGFP caused by GRK-1-93 was confirmed by immunohistological staining of the cones (the arrows in Figures **6A** to **C**).

### Example 2 Selection of gRNA pair

Assuming to use saCas9 (3.2 kb), a donor DNA was designed to treat a Pde6 ^{cpfl1/cpfl1}Gnat1 ^{IRD2/IRD2} mouse with inherited retinal degeneration due to a point mutation in Gnat1 gene. IRD2 mutation of Gnat1 is a homozygous deletion of 59 base pairs in the fourth intron, which inhibits protein expression in the rods comprising about 75% of the mouse retinal cells. Thus, the platform was used to correct this mutation.

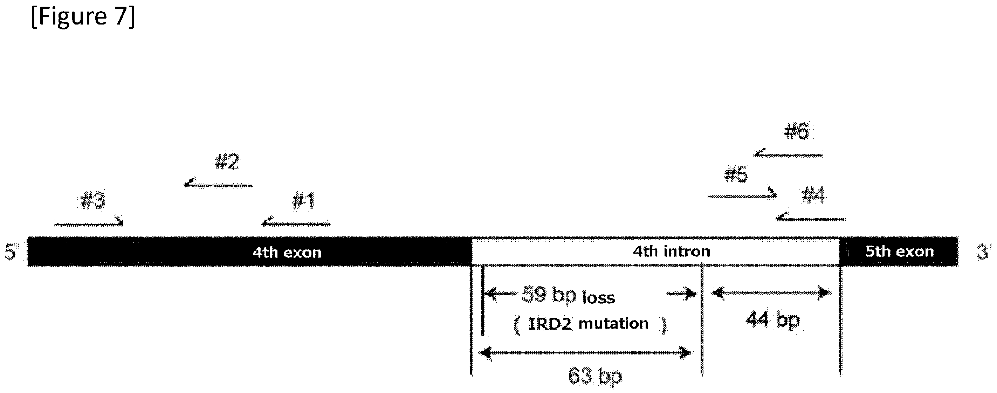

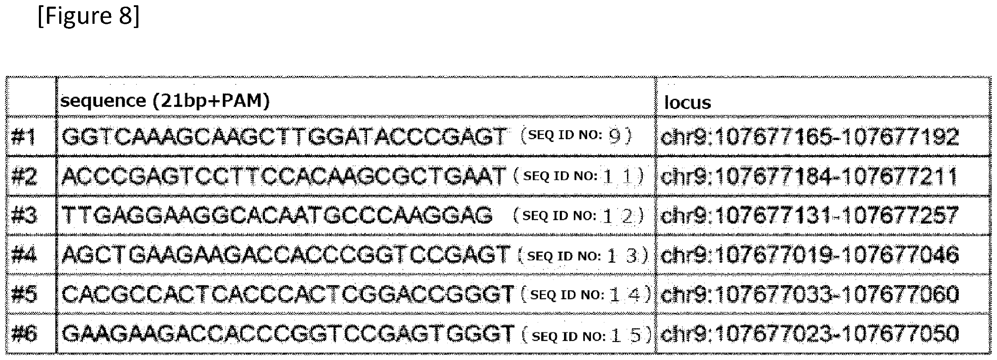

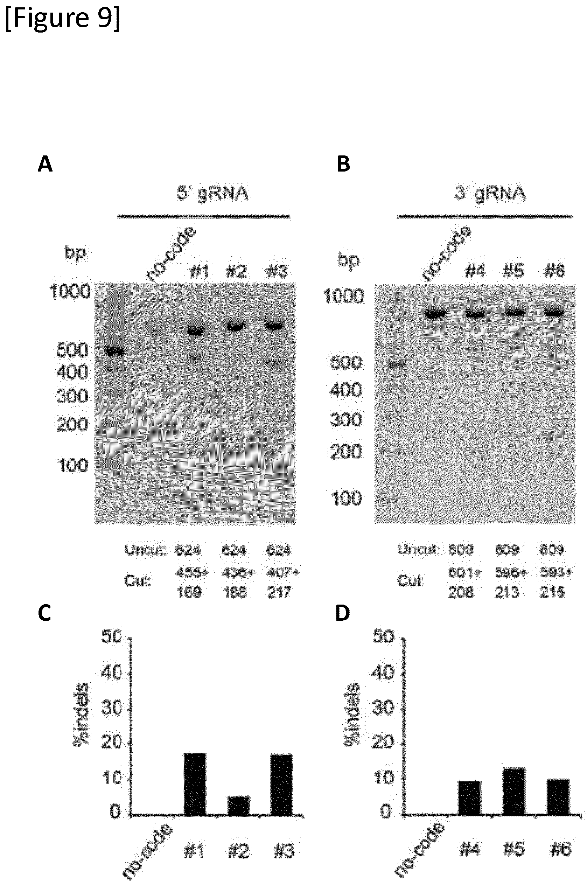

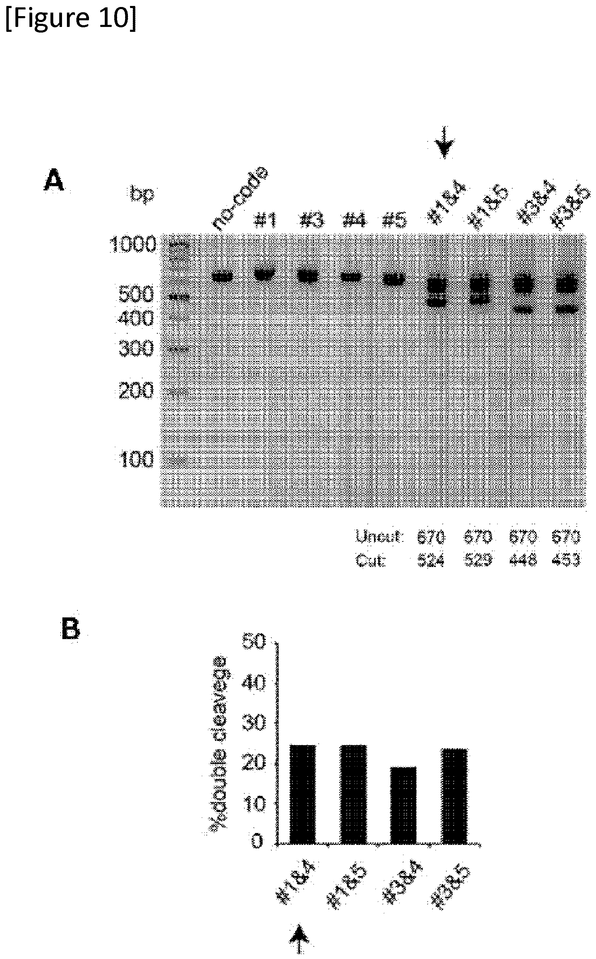

6 gRNAs designed adjacent to the mutation were assessed with a T7 endonuclease 1 assay (Figures **7** to **9**) and a pair of gRNAs (gRNA: 21bp + PAM: 6bp) comprising number 1 (SEQ ID NO: 1) and number 4 (SEQ ID NO: 11) that excised the mutation most efficiently was selected (Figure **10**).

### Example 3 Design of All-in-one AAV vector

Similar to the AAV vector shown in Figure **4** of Test 1, an AAV vector including a first gRNA target sequence (the first gRNA targets a sense strand), a first PAM sequence, a first microhomology arm, a desired nucleic acid, a second microhomology arm, a second PAM sequence (which is a complementary sequence since an antisense strand is targeted), a second gRNA target sequence (the second gRNA targets an antisense strand), a cell-specific promoter (GRK1-93p promoter in the drawing), a sequence encoding a Cas9 nuclease (SaCas9 in the drawing), a first RNA polymerase III promoter (U6 in the drawing), a sequence encoding a first gRNA recognizing a first gRNA target sequence (gRNA-1 in the drawing), a Scaffold sequence, a second RNA polymerase III promoter (U6 in the drawing), a sequence encoding a second gRNA recognizing a second gRNA target sequence (gRNA-2 in the drawing) and a Scaffold sequence was constructed (Figures **11A** to **E** and Figure **12**). However, a mutation of up to several base pairs was designed in the gRNA target site adjacent to the donor sequence to prevent the site from being excised again after successful mutation replacement.

### (Result)

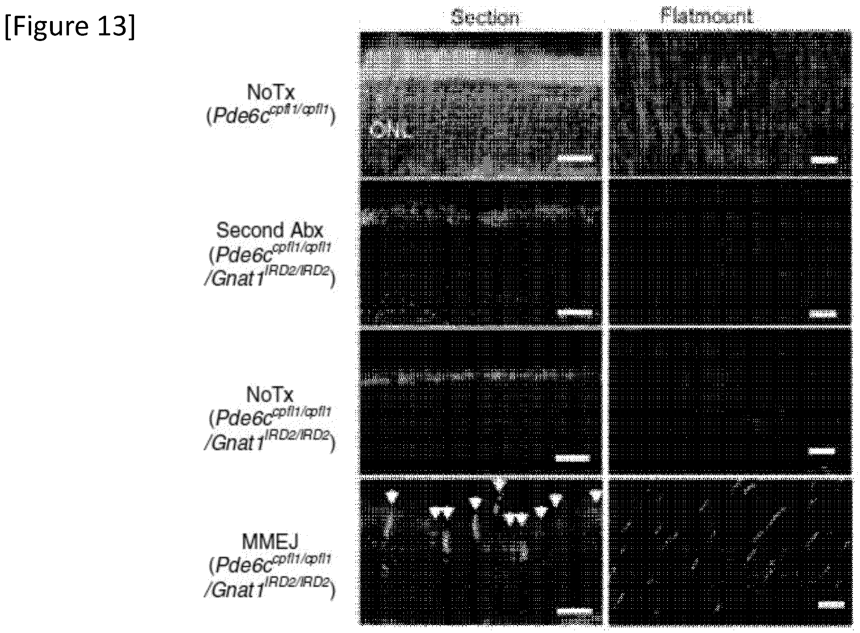

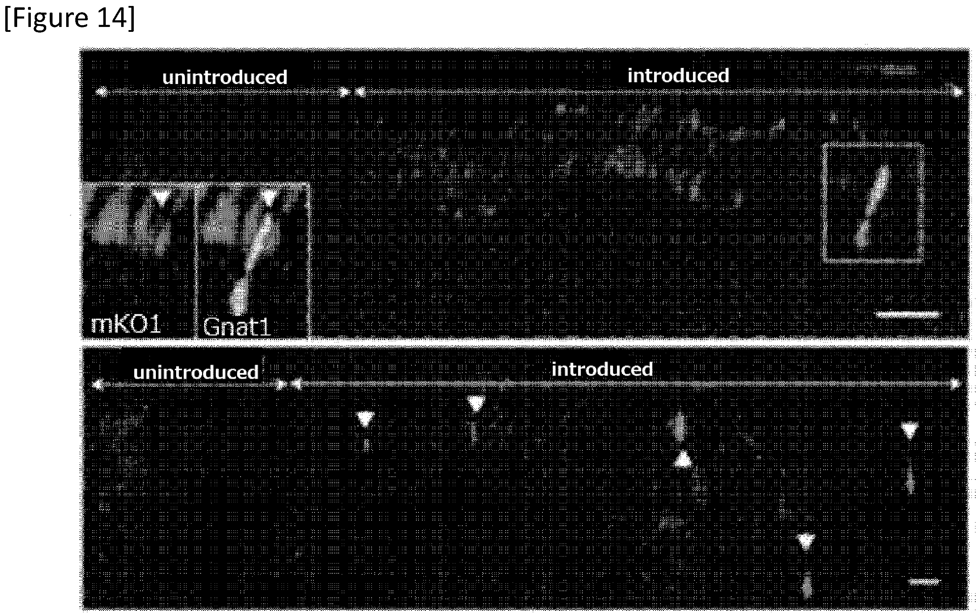

According to the tissue staining after 6 weeks, scattered GNAT1-positive photoreceptor cells were observed (Figure 13), indicating that genome editing was successful. By injecting a modified MMEJ vector tagged with SaCas9 expression by a fluorescent reporter (Figure **11B**), GNAT1 immunoreactivity was observed exclusively in the cells and retinal region with the reporter expression (Figure **14**), suggesting a causal relationship between SaCas9 and GNAT1 expression. According to the tissue staining, there was no sign of acceleration of cone degeneration (data now shown).

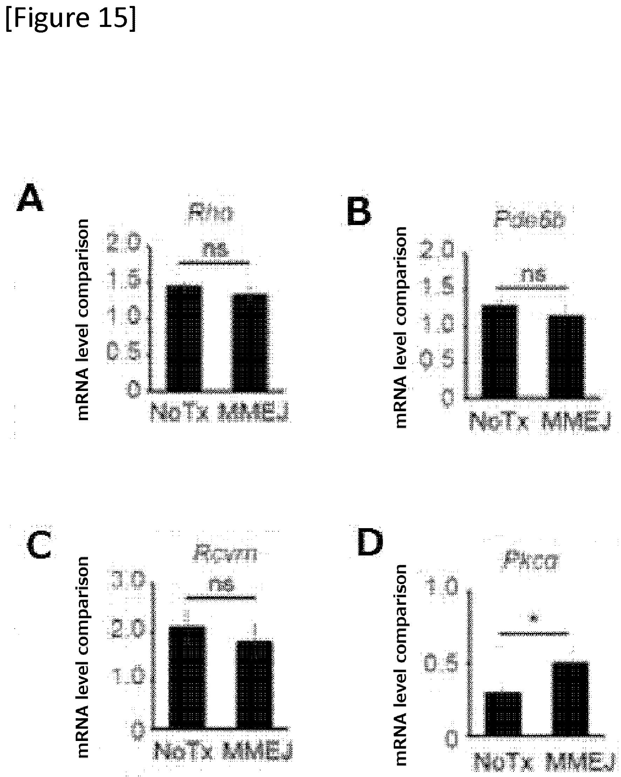

Next, the effect of Gnat1 mutation replacement on mRNA expression of a related gene was examined. While there was no change in the expression of Rho (Figure **15A**) and the expression of Pde6b (Figure **15B**) acting in cooperation with Gnat1 in rod phototransduction as well as in the expression of Rcvn (Figure **15C**) which is a marker of both the rod and the cone compared to an untreated blind mouse, the expression of Pkcα (Figure **15D**), which is a marker of the rod bipolar cells, was reduced to 29.3% of a control. This nearly doubles to 50% after the treatment, which shows that the treated rods interact with the downstream bipolar cells.

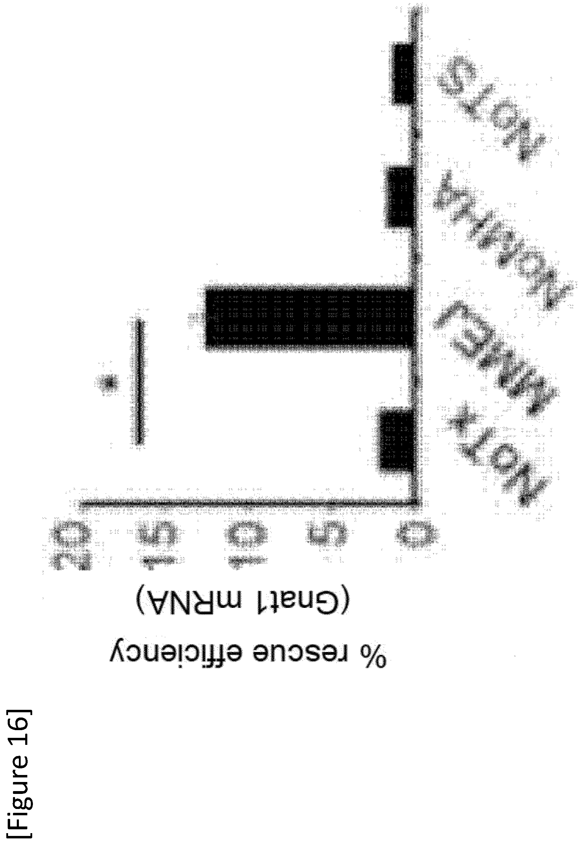

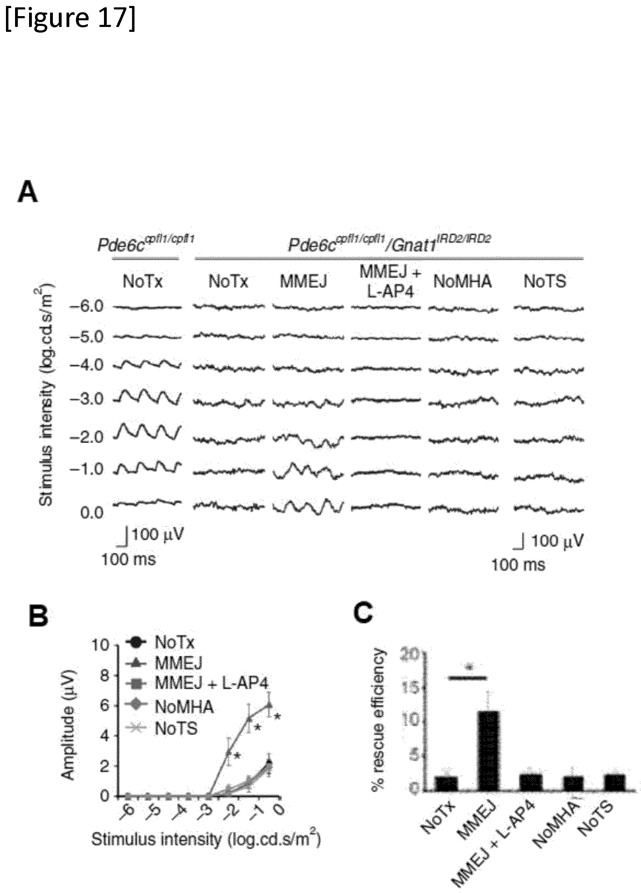

The absolute editing efficiency derived from Gnat1 mRNA expression was about 12.7%. When the gRNA target site adjacent to the MHA or donor sequences was removed from the prototype MMEJ vector (Figures **11C** and **D**), the efficiency dramatically decreased (Figure **16**). This matches with mutation replacement by MMEJ. Furthermore, according to the test using 6-Hz flicker electroretinogram (ERG), which reflects the number of functional photoreceptors (Figure **17**), restoration of the reaction to about 11.2% of the control mouse after MMEJ mutation replacement was demonstrated (Figure **17C**).

### Example 4 On-target sequencing analysis after MMEJ mutation replacement therapy

The inventors of the present invention carried out PCR sequence analysis of the on-target site in vitro and in vivo. In the in vitro analysis, genome-edited clones amplified from mouse Neuro2A cells after introduction of the mutation substitution vector were subjected to on-target sequencing analysis. In the in vitro analysis, genome-edited clones amplified from the retina after mutation replacement therapy of Pde6 ^{cpfl1/cpfl1}Gnat1 ^{IRD2/IRD2} mice were subjected to on-target sequencing analysis. On-target sequencing analysis was carried out at 1M and 3M after MMEJ administration.

### (Result)

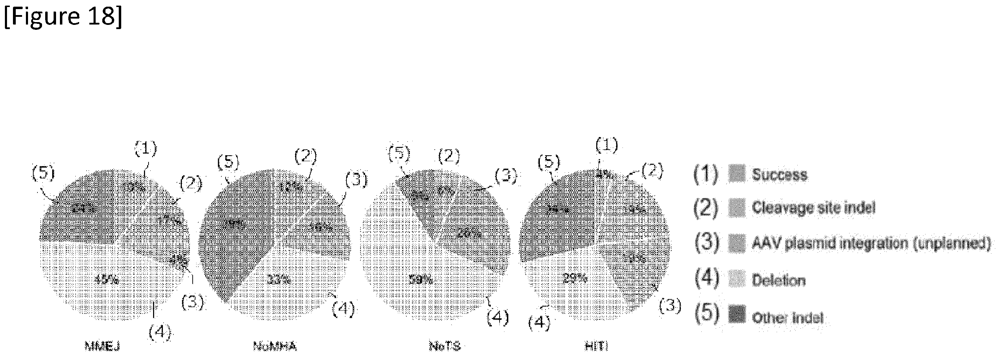

Figure **18** shows in vitro on-target sequencing analysis results. The total number of clones sequenced in this experiment for MMEJ, NoMHA, NoTS and HITI was 70, 67, 84 and 77. The design of each vector is as shown in Figure 11. According to the in vitro analysis, the success rate after MMEJ mutation replacement was shown to be 10.3%.

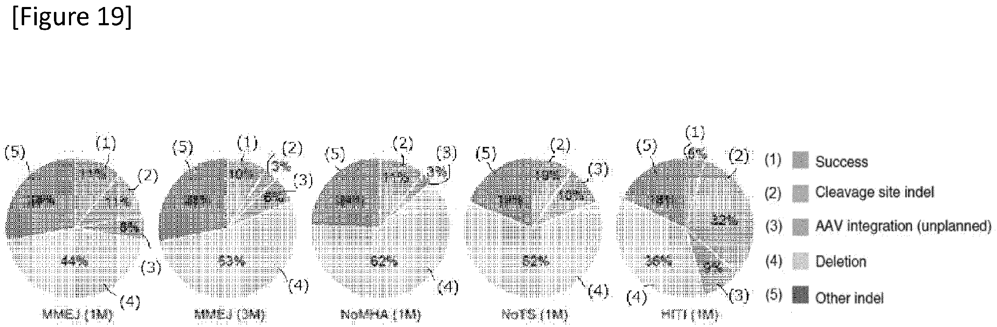

Figure **19** shows in vivo on-target sequencing analysis results. The total number of clones sequenced in this experiment for MMEJ (1M), MMEJ (3M), NoMHA (1M), NoTS (M) and HITI (1M) were 57, 70, 67, 71 and 86. Similarly, in vivo, the success rate of in vivo mutation replacement of the genome-edited rod was 11.1% and 4.5% in the MMEJ method and the HITI method, respectively, 1M after the administration of MMEJ vector. The on-target sequencing analysis and mRNA analysis carried out 3M after administration of MMEJ vector provided the same result as those for 1M after administration (data not shown).

Both in vitro and in vivo analyses did not succeed in mutation replacement with an MMEJ vector with no MHA or gRNA target site.

### Example 5 Off-target analysis

Next, off-target analysis was carried out by sequencing based on the T7E1 assay and PCR of 14 predicted sites shown in Table 1 below. The base sequences of sites OT1-1 to OT1-7 and OT4-1 to OT4-7 are SEQ ID NOs: 16 to 29, respectively.

### (Result)

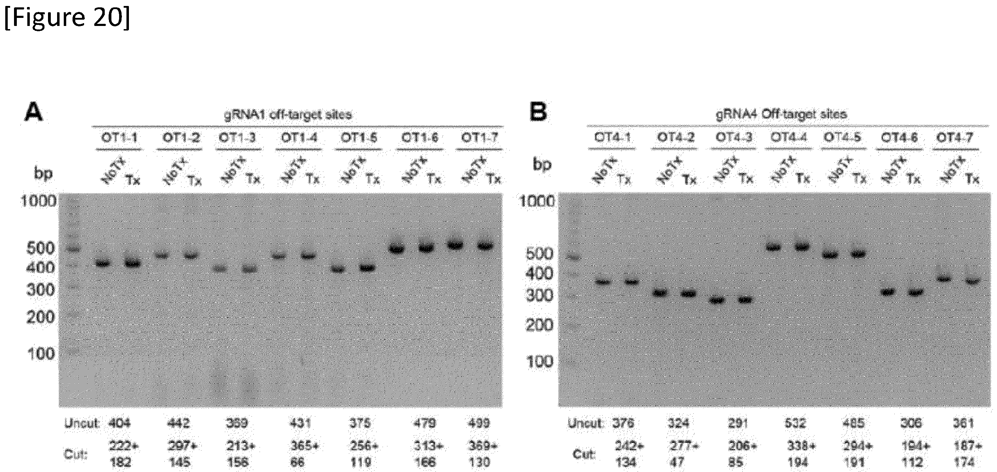

The event of mutation was not detected in the retina collected 1M after injection of MMEJ vector (Figure **20A** and **B**). In these sites, an off-target event did not occur even upon genomic sequencing of 4 retinas of 4 mice 1M after injection of MMEJ vector and 3 retinas of 3 mice 4M after injection of MMEJ vector (data not shown). In Table 1, the CFD score represents the possibility of inducing off-target DNA damage. The number of the sequenced clones and mutations that were found (all were zero) are shown as the denominator and numerator of the "off-target clone" row, respectively. OT: off-target, Tx: T7E1 treatment, NoTx: untreated.

### [Table 1]

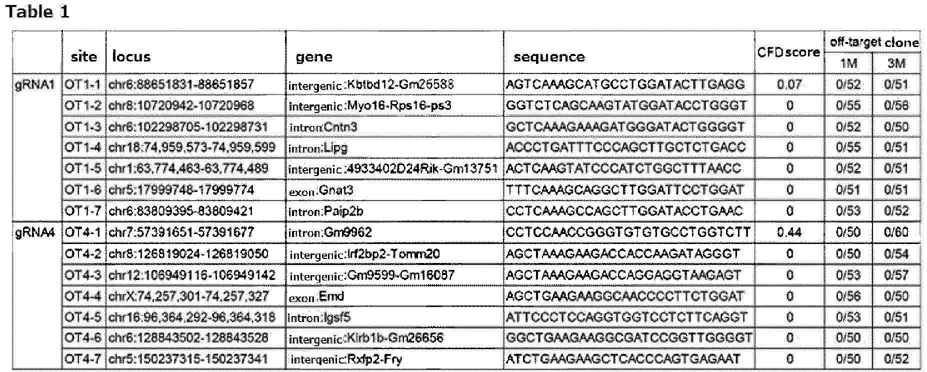

### Example 6 Gnat1 rescue

Therapeutic effect of MMEJ-mediated mutation replacement was examined. The MMEJ vector was injected to a Pde6 ^{cpfl1/cpfl1}Gnat1 ^{IRD2/IRD2} mouse and light sensitivity was measured at the visual cortex by fVEP in the same manner as in Example 4.

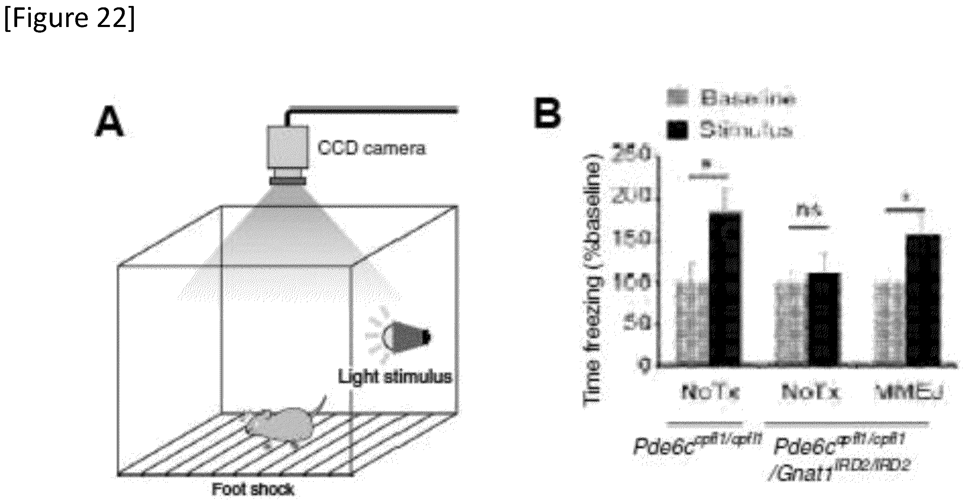

In addition, in the fear conditioning test of a mouse (Figure **22A**, Nat Commun. 2015 Jan 23; 6: 6006), freezing time of before (Baseline) and during (Stimulus) exposure to fear-conditioned light was measured in mice treated with an MMEJ vector (N=9) and untreated mice (N=6).

Furthermore, the pattern visual evoked potential (pVEP) was measured and visual acuity was measured by projecting rotating vertical gratings on the monitor around the mouse placed on a table in a box and by imaging the movement of the mouse with a CCD camera.

### (Result)

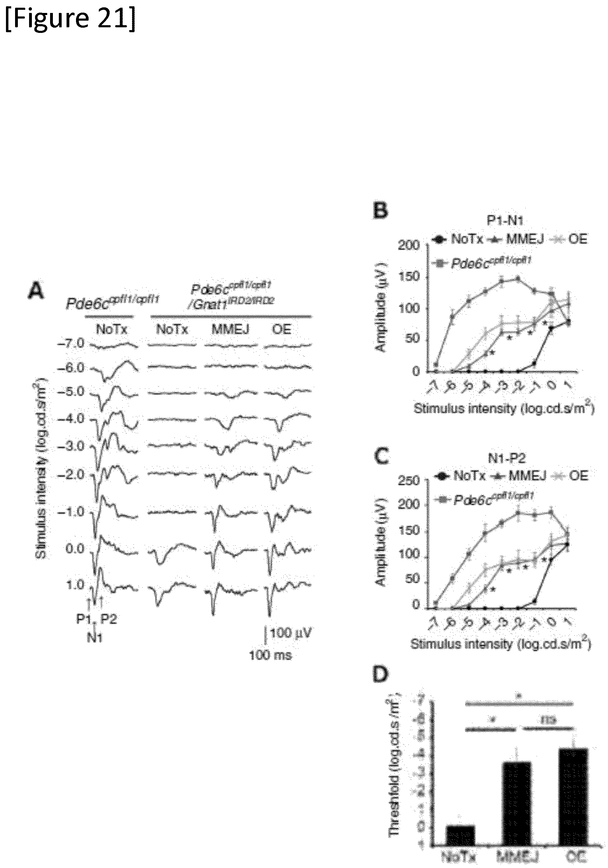

Surprisingly, the light sensitivity measured at the visual cortex contralateral to the treated eye was improved by about 10000-fold, which was similar to the effect of gene supplementation that rescued 70% of photoreceptors and showed a greater ERG response (Figures **21A** to **D**). Furthermore, the pattern visual evoked potential (pVEP) was also shown to increase in amplitude after treatment with the MMEJ vector (data not shown).

In a fear conditioning test of a mouse, a change in light-induced behavior was also recognized in a mouse after MMEJ vector administration (Figure **22B**), which was consistent with improvement in light sensitivity.

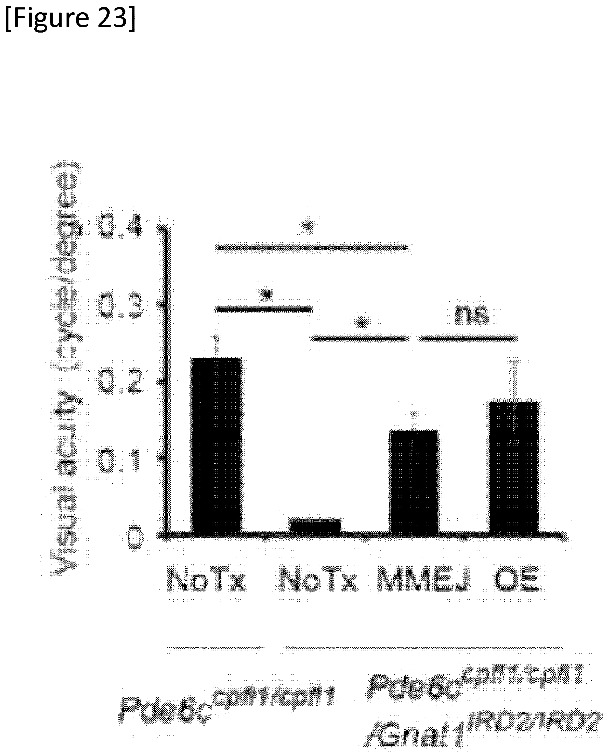

Figure **23** is a result of an experiment on oculomotor reaction. The threshold (visual acuity) of visual spatial resolution measured by oculomotor reaction was recovered to 59.1% of a control mouse in mice treated with the MMEJ vector, which was equivalent to the effect of gene supplementation (Figure **23**).

### Example 7 MMEJ-mediated mutation replacement in human retina dystrophy model mouse

An MMEJ-mediated mutation replacement was used to treat a two-month old Gnat1 ^{IRD2/IRD2} mouse (Carrigan, M., et al., Br. J. Ophthalmol. 100, 495-500 (2016)), which retained cone function and served as a model of human retina dystrophy. In the initial process of this disease, a patient will undergo severe reduction in light sensitivity, but the visual acuity will be retained.

### (Result)

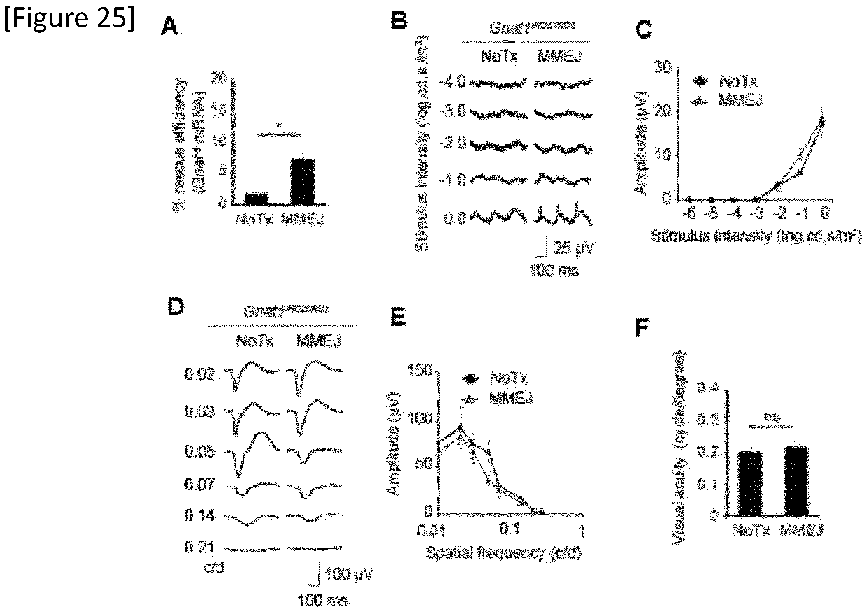

According to histological analysis, scattered GNAT1-positive photoreceptor cells were shown in a treated mouse (Figure **24A**). RT-PCR measurement showed that the absolute editing efficiency is about 7.2% (Figure **25A**). fVEP analysis showed about 1000-fold increase in sensitivity to light (Figures **24B** to **D**). This was behaviorally confirmed in the fear-conditioned test (Figure **24E**).

However, this improvement in retinal function could not be separated from the existing cone function in the ERG test, and visual acuity, pVEP test and oculomotor reaction test showed no improvement (Figures **25B** to **F**). These results show that the therapeutic effect of the present invention of the inventors has been extended to an animal model of a human disease.

The above-described tests show that mutant replacement genome editing with a single AAV vector achieves a remarkable improvement in light sensitivity and visual acuity, which is comparable to gene supplementation. This opens the way to treating loss-of-function mutations in a larger gene where a conventional gene replacement method and NHEJ-based gene editing cannot be applied.

### Test 3

### Application of MMEJ-mediated mutation replacement to Pde6b gene mutation

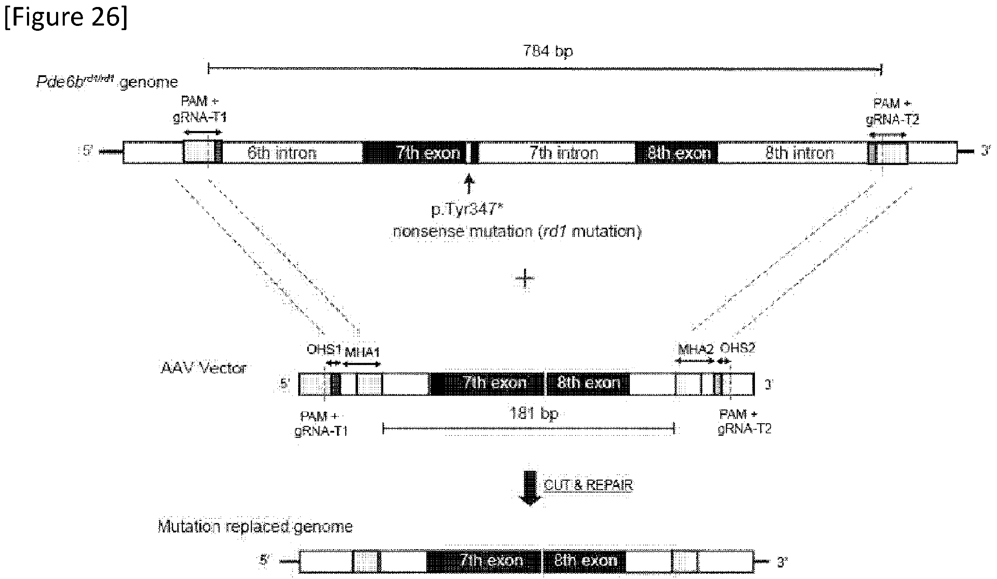

In this test, as shown in Figure **26**, an AAV vector was designed to treat mutation in exon 7 and exon 8 and the effect was examined in vitro. Specifically, the AAV vector targeted a homozygous rd1 mutation at the Pde6e locus (p.Y347* of the 7th exon) that causes severe retinal degeneration. The target genome is cleaved from the mouse genome and the AAV vector at the adjacent gRNA target site (shown by the dotted line in Figure **26**) by the following two gRNAs and SaCaS9. A donor sequence was made so that mutation in exons 7 and 8 could be treated while delivering the vector as a single AAV vector. At the same time, the sequences of both ends of the donor sequence adjacent to the microhomology arm were rendered different from the wild type so that the successfully incorporated donor sequence would not be excised again by SaCas9. The donor template (SEQ ID NO: 32) was inserted in the genome by MMEJ. In addition, on-target sequence analysis was carried out using genome edited clones amplified from a mouse Neuro2A cell after the introduction of the genome editing vector.
gRNA-1 sequence
   TCCAGAGGCCAACTGAAGTCAGGAGT (SEQ ID NO: 30)
gRNA-2 sequence
   TTAGCTGGCTACTAAGCCTGTGGAAT (SEQ ID NO: 31)

The underlines are PAM sequence
Pde6b donor template sequence

TCCAGAGGCCAACTGAAGTCAGGAGTGTCTCCAGAGGCCAACTGAACTAACTTCGACCT
CTGTTCTTTTCCCACAGCACACCCCCGGCTGATCACTGGGCCCTGGCCAGTGGCCTTCC
AACCTACGTAGCAGAAAGTGGCTTTATCTGTAACATCATGAATGCTTCAGCTGATGAAA
TGTTCAACTTTCAGGTAATCTGCCTACCCATTTAATGAAACTGCATTTTAGCTTAGTAG
CCAGCTAATCTATTCCACAGGCTTAGTAGCCAGCTAA
(SEQ ID NO: 32)

### (Result)

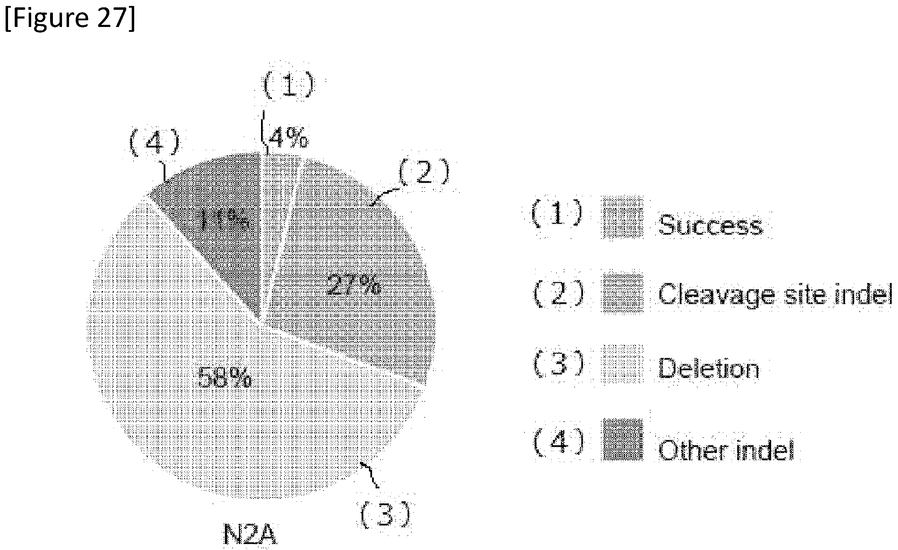

Figure 27 shows an on-target sequencing analysis result. According to the analysis, MMEJ mutation replacement displayed a success rate of 4%.
**The current disclosure is also exemplified by the following:**
**[Item 1]**
   An adeno-associated virus (AAV) vector for inserting a desired nucleic acid into a nucleic acid in a cell,
   wherein the nucleic acid in the cell comprises a region consisting of a first nucleotide sequence and a region consisting of a second nucleotide sequence in order in a direction from a 5' end to a 3' end,
   wherein the vector comprises a first gRNA target sequence, a region consisting of a first nucleotide sequence, the desired nucleic acid, a region consisting of a second nucleotide sequence, a second gRNA target sequence, a cell-specific promoter, a sequence encoding a Cas9 nuclease, an RNA polymerase III promoter, a sequence encoding a first gRNA recognizing the first gRNA target sequence and a sequence encoding a second gRNA recognizing the second gRNA target sequence,
   wherein the vector yields a nucleic acid fragment comprising a region consisting of a first nucleotide sequence, the desired nucleic acid and the region consisting of the second nucleotide sequence by the Cas9 nuclease,
   wherein a first nucleotide sequence in the nucleic acid in the cell and a first nucleotide sequence in the vector are linked by a microhomology-mediated joining and a second nucleotide sequence in the nucleic acid in the cell and a second nucleotide sequence in the vector are linked by a microhomology-mediated joining, thereby inserting the desired nucleic acid between the region consisting of the first nucleotide sequence and the region consisting of the second nucleotide sequence into the nucleic acid in the cell.
[**Item** 2]
   The vector of **item** 1, wherein the nucleic acid in a cell has a target nucleic acid sequence replaced with the desired nucleic acid between the region consisting of the first nucleotide sequence and the region consisting of the second nucleotide sequence,
   has a first gRNA target sequence recognized by the first gRNA of the AAV vector and a first PAM sequence between the region consisting of the first nucleotide sequence and the target nucleic acid sequence, and
   has a second gRNA target sequence recognized by the second gRNA of the AAV vector and a second PAM sequence between the target nucleic acid sequence and the region consisting of the second nucleotide sequence.
[**Item** 3]
   The vector of **item** 2, wherein the vector satisfies any of the following (i) to (iv).
   (i) not having one or both of a sequence which is the same as the first gRNA target sequence and a sequence which is the same as the second gRNA target sequence between the region consisting of the first nucleotide sequence and the region consisting of the second nucleotide sequence,
   (ii) when there is a sequence which is the same as the first gRNA target sequence between the region consisting of the first nucleotide sequence and the region consisting of the second nucleotide sequence, the first PAM sequence being mutated so as to avoid cleavage of the first gRNA target sequence by a Cas9 nuclease,
   (iii) when there is a sequence which is the same as the second gRNA target sequence between the region consisting of the first nucleotide sequence and the region consisting of the second nucleotide sequence, the second PAM sequence being mutated so as to avoid cleavage of the second gRNA target sequence by a Cas9 nuclease, or
   (iv) both (ii) and (iii).
[**Item** 4]
   The vector of any one of **items** 1 to 3, wherein the nucleic acid in the cell has a mutation type base between the region consisting of the first nucleotide sequence and the region consisting of the second nucleotide sequence, has a normal type base in which the desired nucleic acid of the vector corresponds to the mutation type base, wherein the mutation type base of the nucleic acid in the cell is replaced with the normal type base.
[**Item** 5]
   The vector of any one of **items** 1 to 4, wherein a length of the cell-specific promoter is 200 bases or less.
[**Item** 6]
   The vector of any one of **items** 1 to 5, wherein the cell-specific promoter is a promoter selected from rhodopsin kinase promoter, RPE65 promoter, Best1 promoter, mGluR6 promoter, cone arrestin promoter, CRALBP1 promoter, Chx10 promoter, rhodopsin promoter, cone opsin promoter, recoverin promoter, synapsin I promoter, myelin basic protein promoter, neuron-specific enolase promoter, calcium/calmodulin-dependent protein kinase II (CMKII) promoter, tubulin α I promoter, platelet-derived growth factor β chain promoter, glial fibrillary acidic protein (GFAP) promoter, L7 promoter and glutamic acid receptor delta 2 promoter; or a promoter having consecutive 50 to 150 base sequence of the promoter; or a promoter consisting of a base sequence that is 90% or more identical with the 50 to 150 base sequence.
[**Item** 7]
   The vector of any one of **items** 1 to 6, wherein a length of a base of the first nucleotide sequence in the vector is 5 to 40 and a length of a base of the second nucleotide sequence in the vector is 5 to 40.
[**Item** 8]
   A kit for inserting a desired nucleic acid into a nucleic acid in a cell, comprising the vector of any one of items 1 to 7.
**[Item** 9]
   A method of inserting a desired nucleic acid into a nucleic acid in a cell, comprising the step of introducing the vector of any one of **items** 1 to 7 to a cell.
**[Item** 10]
   A method of manufacturing a cell comprising a desired nucleic acid, comprising:
   the step of introducing the vector of any one of **items** 1 to 7 to a cell; and
   the step of inserting a desired nucleic acid into the nucleic acid in the cell.
[**Item** 11]
   A method for treating a disease in vitro or in a non-human animal, comprising:
   the step of introducing the vector of any one of **items** 1 to 7 to a cell; and
   the step of inserting a desired nucleic acid into the nucleic acid in the cell.
[**Item** 12]
   The method of **item** 11, wherein the cell is an ocular cell.
[**Item** 13]
   A therapeutic composition for treating a disease, comprising the vector of any one of **items** 1 to 7.

## Claims

1. A method of manufacturing an adeno-associated virus (AAV) vector for inserting a desired nucleic acid into a nucleic acid in a cell, comprising:
respectively arranging a first gRNA target sequence, a first PAM sequence, a first nucleotide sequence, the desired nucleic acid, a second nucleotide sequence, a second PAM sequence and a second gRNA target sequence in order in a direction from a 5' end to a 3' end to cause a microhomology-mediated joining (Microhomology-mediated end-joining, MMEJ) with a genomic nucleic acid in the cell; and
arranging the vector to further comprise a promoter specific to the cell, a sequence encoding a Cas9 nuclease, a first RNA polymerase **III** promoter, a sequence encoding a first gRNA recognizing the first gRNA target sequence, a Scaffold sequence, a second RNA polymerase III promoter, a sequence encoding a second gRNA recognizing the second gRNA target sequence and a Scaffold sequence in order in a direction from a 5' end to a 3' end;
wherein the vector is configured to comprise a cleavage site that yields, by the Cas9 nuclease, a nucleic acid fragment comprising the desired nucleic acid, the first nucleotide sequence, and the second nucleotide sequence; and
wherein the length of the promoter specific to the cell is 500 bases or less.

2. The method of claim 1, comprising introducing, when there is a sequence which is the same as the first and/or second gRNA target sequence between the first nucleotide sequence and the second nucleotide sequence, a mutation in the first and/or second gRNA target sequence so as to avoid cleavage of the first and/or second gRNA target sequence present between the first nucleotide sequence and the second nucleotide sequence by the Cas9 nuclease.

3. The method of claim 1 or 2, comprising introducing, when there is a sequence which is the same as the first and/or second gRNA target sequence between the first nucleotide sequence and the second nucleotide sequence, a mutation in a first PAM sequence adjacent to the first gRNA target sequence and/or a second PAM sequence adjacent to the second gRNA target sequence so as to avoid cleavage of the first and/or second gRNA target sequence present between the first nucleotide sequence and the second nucleotide sequence by the Cas9 nuclease.

4. The method of any one of claims 1 to 3, wherein the promoter specific to the cell is a promoter selected from rhodopsin kinase promoter, RPE65 promoter, Best1 promoter, mGluR6 promoter, cone arrestin promoter, CRALBP1 promoter, Chx10 promoter, rhodopsin promoter, cone opsin promoter, recoverin promoter, synapsin I promoter, myelin basic protein promoter, neuron-specific enolase promoter, calcium/calmodulin-dependent protein kinase II (CMKII) promoter, tubulin α I promoter, platelet-derived growth factor β chain promoter, glial fibrillary acidic protein (GFAP) promoter, L7 promoter and glutamic acid receptor delta 2 promoter; or a promoter having consecutive 50 to 150 base sequence of the promoter; or a promoter consisting of a base sequence that is 90% or more identical with the 50 to 150 base sequence.

5. The method of any one of claims 1 to 4, wherein the number of bases in the first and/or second nucleotide sequence is 5 to 40.

6. A vector manufactured by the method of any one of claims 1 to 5.

7. An adeno-associated virus (AAV) vector for inserting a desired nucleic acid into a nucleic acid in a cell, comprising:
a first gRNA target sequence, a first PAM sequence, a first nucleotide sequence, the desired nucleic acid, a second nucleotide sequence, a second PAM sequence and a second gRNA target sequence arranged in order in a direction from a 5' end to a 3' end, wherein the first nucleotide sequence and the second nucleotide sequence cause a microhomology-mediated joining (Microhomology-mediated end-joining, MMEJ) with a genomic nucleic acid in the cell; and
a promoter specific to the cell, a sequence encoding a Cas9 nuclease, a first RNA polymerase III promoter, a sequence encoding a first gRNA recognizing the first gRNA target sequence, a Scaffold sequence, a second RNA polymerase **III** promoter, a sequence encoding a second gRNA recognizing the second gRNA target sequence and a Scaffold sequence arranged in order in a direction from a 5' end to a 3' end;
wherein the vector is configured to comprise a cleavage site that yields, by the Cas9 nuclease, a nucleic acid fragment comprising the desired nucleic acid, the first nucleotide sequence, and the second nucleotide sequence; and
wherein the length of the promoter specific to the cell is 500 bases or less.

8. The vector of claim 7, which is for treating a disease in a subject.

9. A therapeutic composition for use in treating a disease, comprising a vector manufactured by the method of any one of claims 1 to 5.

10. A promoter comprising the nucleotide sequence set forth in SEQ ID NO: 4.

11. An adeno-associated virus (AAV) vector comprising the promoter of claim 10.

12. A method for retina-specific transcription of a gene, comprising using the promoter of claim 10 as a promoter for the transcription of the gene or the adeno-associated virus (AAV) vector comprising the promoter of claim 10.
